# EUROPEAN PATENT SPECIFICATION

(11) **EP 4 021 533 B1**
(45) Date of publication and mention of the grant of the patent: **01.10.2025**
(21) Application number: 19787489.4
(22) Date of filing: 26.08.2019
(51) Int. Cl.: A61M 5/155, A61M 5/168, A61M 5/00, A61M 5/142

(54) **A CARTRIDGE FOR MIXING A LIQUID INTENDED FOR INTRACORPOREAL USE**
KARTUSCHE ZUM MISCHEN EINER FLÜSSIGKEIT ZUR INTRAKORPORALEN VERWENDUNG
CARTOUCHE POUR MÉLANGER UN LIQUIDE DESTINÉ À UNE UTILISATION INTRACORPORELLE

(43) Date of publication of application: 06.07.2022
(73) Proprietor: Solstice Pharmaceuticals B.V., 7545 PN Enschede (NL)
(72) Inventor: VAN HOEVE, Willem, 7522 HC Enschede (NL); GIANNASI, Katharine Jennifer, 7522 HC Enschede (NL); BRAMER, Koen Joannes Jonathan, 7671 PH Vriezenveen (NL); DE VARGAS SERRANO, Miguel, 7512 HK Enschede (NL); LUCASSEN, Koen Rik, 7597 NE Saasveld (NL); VAN DER PLAS, Renzo Bouwe, 7312AN Apeldoorn (NL); PENTERMAN, Roel, 7555 VX Hengelo (NL); EILANDER, Jan Wiebe, 7558 MA Hengelo (NL); HEIJDENS, Erik Jan Herman, 7577 BT Oldenzaal (NL); VAN VOORTHUIZEN, Vivian Anoek, 7521 ZD Enschede (NL)
(74) Representative: V.O.
(86) International application number: PCT/NL2019/050547
(87) International publication number: WO 2021/040513

(56) References cited:
- WO-A2-2012/099898
- US-A1- 2011 282 381

## Description

The present invention is related to a cartridge. More in particular, the present invention relates to a cartridge by which a liquid held in the cartridge can be mixed, within the cartridge, with a pressurized gas. The present invention further relates to a cartridge system comprising such a cartridge and a device in which the cartridge can be releasably inserted, wherein the device is configured for providing pressurized gas(ses) to be used as propellant for moving the liquid inside the cartridge and/or to be used as gas to be mixed. The present invention also relates to a device for releasably receiving the abovementioned cartridge.

When a therapeutic or diagnostic liquid is to be used intracorporeally, it is essential that the sterility of the liquid can be ensured. When such liquid can be used without additional processing steps such as mixing the liquid with another liquid, it is possible to supply the liquid in a sterile container intended for one person. Alternatively, a larger source of sterile liquid can be used from which the required quantity can be drawn. However, in this latter case, strict requirements apply to the environment in which the reservoir is placed and the manner in which the desired quantities are obtained therefrom. This latter aspect makes the use of larger reservoirs impractical for environments such as hospitals or clinics.

A different situation however occurs when the liquid needs to be processed before use. For example, the therapeutic or diagnostic substance to be administrated to the patient requires a personalized composition or dosage. Alternatively, it may happen that the therapeutic or diagnostic substance is inherently instable and must be administrated quickly after preparation. In such situations, ensuring sterility of the end product is more difficult.

US 2011/282381 A1, discloses, according to its abstract, a surgical device comprising a housing, a bioprocessing module, an end effector, and a pumping device. The bioprocessing module comprises media reservoirs, fluid conduits in communication with the reservoirs, and a port through which media is expelled. The end effector is configured for insertion into a lumen and delivering a tissue repair composition into that lumen. The end effector has at least one fluid conduit extending therethrough and at least one orifice in communication with the fluid conduit. The fluid conduit in the first end effector is in fluid communication with the port on the bioprocessing module. The pumping device is operable to urge a tissue repair composition comprising at least a portion of the contents of the media reservoirs through the fluid conduit of the first end effector such that the tissue repair composition is expelled from the at least one orifice.

WO 2012/099898 A2, discloses, according to its abstract, a cartridge for use with a beneficial agent delivery device having a fillable reservoir. The cartridge includes a reconstitution, unit dose package having first and second compartments and a fitment. The first compartment contains at least a first constituent of the beneficial agent. The second compartment contains at least a second constituent of the beneficial agent. The fitment is disposed on the package for interfacing the package to the fillable reservoir. An object of the present invention is to provide a solution to the abovementioned problem.

According to the invention, this object is achieved with a cartridge as defined in claim 1. This cartridge comprises a cartridge body, one or more gas inlets formed in the cartridge body, and a fluid storage system formed in the cartridge body and comprising a fluid storage unit being configured for holding a liquid and for outputting said liquid in response to a pressurized gas being supplied to the fluid storage unit through a gas inlet among the one or more gas inlets by using the supplied gas as a propellant.

The cartridge further comprises a mixing unit arranged in or mounted to the cartridge body and being in fluid communication with the fluid storage unit using a fluid channel formed in the cartridge body. The mixing unit is configured for mixing the liquid outputted from the fluid storage unit with a pressurized gas received through a gas inlet among the one or more gas inlets.

With the cartridge of the invention it becomes possible to hold the liquid to be mixed in a sterile manner inside the cartridge. Moreover, pressurized gas is used to control the mixing process by using this gas as propellant. Hence, the liquid can be mixed within the cartridge thereby avoiding the possible non-sterile transport of liquid between the mixing unit and a reservoir of liquid. The only fluids that are exchanged with the cartridge are gaseous, and gases can be kept sterile in a much more convenient manner than liquids.

As the liquid can be kept in the fluid storage unit in a sterile manner, it becomes possible to produce and store the cartridges for a considerable period of time prior to administration.

The invention is particularly related to cartridges in which the liquid held in the fluid storage system is intended for intracorporeal use in a human or animal, for example for intravenous use or intra-cavity use. For example, the liquid may comprise or may be used to form a diagnostic agent, such as a contrast agent for medical imaging, or a therapeutic agent, such as a medicament. Nevertheless, the present invention can also relate to other applications wherein the sterility of the liquid to be mixed and the sterility of the mixed product are of importance.

The fluid storage unit comprises a fluid storage unit inlet and a fluid storage unit outlet, wherein the fluid storage unit is configured such that the pressurized gas supplied to the fluid storage unit through the fluid storage unit inlet pushes the fluid in the fluid storage unit through the fluid storage unit outlet. By using a gas, which can be kept sterile relatively easily, as a propellant, the sterility of the liquid can be ensured during transport from the fluid storage unit to the mixing unit. In addition, the fluid storage unit comprises a storage chamber configured for receiving a sealed off container in which the liquid is held in a sterile and sealed off manner, and a liquid reservoir in fluid communication with the storage chamber, wherein the fluid storage unit inlet is connected to one of the storage chamber and the liquid reservoir and wherein the fluid storage unit outlet is connected to the liquid reservoir. The liquid reservoir is configured for collecting liquid that is released from the container after the container has been broken, ruptured, cut, or pierced. The sealed off container may for example comprise a blister package. This sealed off container may be fixedly held in the storage chamber, for example by means of an adhesive. Alternatively, the blister package is inserted into the storage chamber just before use. For example, the blister package may comprise an adhesive strip by which the blister package can be fixed to the cartridge. Such adhesive strip could be covered by a covering strip that is to be removed when the blister package is to be fixed to the cartridge. Hence, the present invention may relate to embodiments wherein the cartridge and sealed off container are two separate items that are sold to the end-user by a single entity or separately by different entities, or the invention may relate to embodiments wherein the sealed off container is fixed to the cartridge before the combination is sold to the end-user.

The liquid reservoir may be formed as a part of the storage chamber. In this case, the liquid that is released from the sealed off container is collected at least partially in the storage chamber itself. Alternatively, the fluid storage unit may comprise a fluid channel formed in the cartridge body connecting the storage chamber and the liquid reservoir. Hence, after the liquid is released, it will be transported through this fluid channel to the liquid reservoir.

The storage chamber may comprise a supporting surface for supporting the sealed off container and at least one protruding pin or needle extending towards the sealed off container for the purpose of puncturing through the sealed off container if sufficient force is exerted onto the sealed off container. The sealed off container may be deformable such that when force is exerted, a wall or segment of the container engages the pin or needle. When sufficient force is applied, the pin or needle will puncture the sealed off container thereby releasing the liquid that is held by that container. It should be noted that the present invention does not exclude other means by which the container may be broken, cut, ruptured, or otherwise opened for the purpose of releasing the liquid. The pin or needle may protrude from the supporting surface. In other embodiments, the pin(s) or needle(s) may be provided at a different surface such that when the container is supported by the support surface and no external force is exerted onto the container, the pin(s) or needle(s) do not engage the container.

In addition, the fluid storage unit may be provided with a protective ring that protrudes away from the cartridge body further than the sealed off container when it is placed in the storage chamber. In this manner, when the cartridge is placed onto a table with the container facing downward towards the table, there will be no significant force that is exerted onto the container.

The gas inlet through which the pressurized gas is received that is to be mixed with the liquid can be the same as the gas inlet through which the pressurized gas is received that is used as propellant by the fluid storage unit. The pressurized gas that is to be mixed with the liquid can be different from the pressurized gas that is used as propellant by the fluid storage unit.

The present invention particularly relates to the combinations of gasses and liquids as described in table 1. **In** this table, (m) indicates that a gas is to be used as a gas to be mixed, and (p) indicates that it is to be used as a propellant. For example, the second combination mentioned in the table indicates that a first gas is used as the pressurized gas to be mixed, and a second gas is used as a propellant for propelling the different liquids, i.e. liquid 1 and liquid 2 held in respective fluid storage units, towards the mixing unit. **In** the fifth combination, a first gas is fed through gas inlet 1 and is used both as a propellant and as gas to be mixed. Other combinations are not excluded.

**Table 1: Possible combinations of gases and liquids.**

| *Gas inlet 1* | *Gas inlet 2* | *Gas inlet 3* | *Fluid storage unit 1* | *Fluid storage unit 2* |
|---|---|---|---|---|
| Gas 1 (m) | Gas 2 (p) | Gas 3(p) | Liquid 1; gas 2 (p) | Liquid 2; gas 3 (p) |
| Gas 1 (m) | Gas 2 (p) | Not used | Liquid 1; gas 2 (p) | Liquid 2; gas 2 (p) |
| Gas 1 (m) | Gas 1 (p) | Not used | Liquid 1; gas 1 (p) | Not used |
| Gas 1 (m) | Gas 2 (p) | Not used | Liquid 1; gas 2 (p) | Not used |
| Gas 1 (m, p) | Not used | Not used | Liquid 1; gas 1 (p) | Not used |

Among these combinations, the invention relates to embodiments wherein the cartridge has a single fluid storage unit, and two gas inlets, wherein a first gas inlet among the two gas inlets is in fluid communication with the mixing unit and wherein a second gas inlet among the two gas inlets is in fluid communication with the fluid storage unit. This corresponds to the third combination in the table above.

The mixing unit can be configured to mix the liquid received from the fluid storage system with a pressurized gas, wherein the mixing unit comprises a microfluidics device configured for generating microbubbles within the liquid that are filled with the pressurized gas. The microfluidics device may comprise two or more substrates, for example glass substrates, in which at least one substrate comprises a structure of grooves made, for example, using wet etching, and which substrates are bonded together, for example using fusing bonding, to form a structure of channels having cross-sectional dimensions in a range of 1-1000 micrometer. The microfluidics device can be configured for generating microbubbles having a diameter below 10 micrometer, and preferably in a range of 2-5 micrometer. Such microbubbles can be used as a contrast agent for ultrasound imaging or for therapeutic application in combination with ultrasound, such as ultrasound directed drug delivery or focused ultrasound therapy. For such application, the pressurized gas to be mixed may comprise one or more gases from the group consisting of SF₆, N₂, CO₂, O₂, H₂, He, Ar, ambient air, and perfluorocarbon gases, such as CF₄, C₂F₆, C₂F₈, C₃F₆, C₃F₈, C₄F₆, C₄F₈, C₄F₁₀, C₅F₁₀, C₅F₁₂ and mixtures thereof. Additionally or alternatively, the liquid received from the fluid storage system may comprise at least one liquid from the group consisting of water, dispersion of lipids, such as phospholipids, or proteins in an aqueous solution, active pharmaceutical ingredients, and/or alcohols. Either the pressurized first gas or the liquid(s) received from the fluid storage system may comprise a surfactant suitable for populating a fluid interface between the pressurized first gas and liquid(s) from the fluid storage system, and to thus encapsulate and stabilize the microbubbles and to thereby prevent the dissolution of the bubbles. The surfactant may, for example, include a film-forming (mixture of) phospholipid(s), e.g. a mixture of DPPC, DSPC, DPPA, and DPPE-MPEG5000.

When used in the abovementioned application for generating a contrast agent for ultrasound imaging, the cartridge preferably comprises a gas inlet for receiving the pressurized gas to be mixed, and a gas inlet for receiving the propellant gas to be used for the single fluid storage unit.

The microfluidics device may comprise a first inlet for receiving the pressurized gas to be mixed, a second inlet for receiving said at least one liquid, and a bubble formation channel for generating the microbubbles based on a flow of the pressurized gas received through the first inlet and a flow of the liquid received through the second inlet. Additionally, the cartridge body may comprise a first opening, a second opening, and a third opening, wherein the first opening is in fluid communication with the gas inlet that receives the pressurized gas to be mixed, and wherein the second opening is in fluid communication with the fluid storage system for the purpose of receiving the liquid. In this case, the microfluidics device is positioned relative to the cartridge such that the first opening is aligned with the first inlet of the microfluidics device, the second opening with the second inlet of the microfluidics device, and the third opening with an outlet of the microfluidics device. The microfluidics device may be fixedly connected to the cartridge body using an adhesive or a monolithic bond. Alternatively, the microfluidics device can be integrally formed with the cartridge body.

The microfluidics device may comprise a flow focusing junction, a first channel connected on one end to the second inlet and on another end to the flow focusing junction, a second channel connected on one end to the second inlet and on another end to the flow focusing junction, and a third channel connected on one end to the first inlet and on another end to the flow focusing junction. The bubble formation channel may be connected to the flow focusing junction. Moreover, the flow focusing junction may be configured to receive a flow of said liquid from two opposing directions via the first and second channels that impinges in a perpendicular manner onto a flow of the pressurized gas to be mixed received via the third channel. The flow of the pressurized gas may be directed from the third channel into the bubble formation channel.

A microfluidics device as described above is known from WO 2013/141695 and WO 2016/118010. The contents of these publications are incorporated herein for all purposes.

The cartridge may comprise an outlet formed in the cartridge body for outputting the liquid and pressurized gas mixed by the mixing unit. This outlet can be formed to allow a Luer taper connection with a syringe.

The cartridge may comprise a buffer reservoir formed in the cartridge body and arranged in between the mixing unit and the outlet, wherein the buffer reservoir has a capacity that exceeds the volume of liquid held in the fluid storage system. Typically, the buffer reservoir is at least 30 percent larger than the volume of liquid held in the fluid storage system.

The cartridge may further comprise a venting hole formed in the cartridge body that is in fluid communication with the buffer reservoir. The venting hole is provided to prevent over-pressure in the buffer reservoir. Furthermore, the venting hole allows the buffer reservoir to be purged with a predefined gas prior to mixing. For example, in the embodiment with the microfluidics device described above, it is possible to purge the buffer reservoir with the same gas as the gas that fills the microbubbles. By having the buffer reservoir filled with this gas, the stability of the microbubbles can be improved, e.g. the dissolution rate of the microbubbles can be reduced compared to embodiments wherein other gaseous media are present in the buffer reservoir. The venting hole further allows pressure equalization in the buffer reservoir when the liquid is extracted from the buffer reservoir, e.g. using a syringe. Without the venting hole, an under-pressure would be generated inside the buffer reservoir that would destroy the microbubbles and complicate the extraction of the liquid inside the buffer reservoir.

The cartridge may further comprise a fluid channel formed in the cartridge body in between the one or more gas inlets and the mixing unit, a fluid channel formed in the cartridge body in between the one or more gas inlets and the fluid storage system, a fluid channel formed in the cartridge body in between the fluid storage system and the mixing unit, a fluid channel formed in the cartridge body in between the mixing unit and the buffer reservoir, and a fluid channel formed in the cartridge body in between the buffer reservoir and the venting hole. These fluid channels may extend essentially in a same fluid channel plane. In some embodiments, the cartridge may comprise a top surface and a bottom surface that are relatively close together and wherein the top surface and bottom surface essentially extend in a plane that is parallel to the fluid channel plane. Such cartridge may be bar shaped.

The cartridge may be configured to be in a first orientation during said mixing, wherein, in the first orientation, a normal to the fluid channel plane is substantially horizontal, the mixing unit is positioned at a lower position relative to the fluid storage system and relative to the gas inlet through which the pressurized gas to be mixed is received, and the fluid channel between the mixing unit and the buffer reservoir outputs in the buffer reservoir at a lower end of the buffer reservoir, preferably at or close to a lowest point of the buffer reservoir in this orientation. Hereinafter, the wording horizontal and vertical will be used as relating to a direction of Earth's gravitational force unless otherwise stated. For example, vertical shall be used to refer to a direction perpendicular to Earth's surface and horizontal to a direction parallel to Earth's surface.

Typically, the gas that fills the microbubbles is heavier than ambient air. When the cartridge is held in the first orientation, and the buffer reservoir has been flushed with this gas prior to mixing, liquid exiting the microfluidics device will, when entering the buffer reservoir, engage this gas and/or the already outputted liquid. In this manner, the stability of the microbubbles can be improved as contact between the outputted liquid and ambient air that may be present in the buffer reservoir can be minimized.

The cartridge can be configured to be in a second orientation e.g. during storage, wherein, in the second orientation a normal to the fluid channel plane is substantially vertical, and the fluid channel between the mixing unit and the buffer reservoir outputs in the buffer reservoir at a position above a liquid-air interface in the buffer reservoir to prevent liquid outputted by the mixing unit into the buffer reservoir to flow back into the fluid channel between the mixing unit and the buffer reservoir when the cartridge is in the second orientation. Hence, after mixing with the cartridge being held in the first orientation, the cartridge may be arranged flat on a table. This configuration generally corresponds to the second orientation. By arranging the fluid channel between the mixing unit and the buffer reservoir in the manner described, it can be achieved that the liquid in the buffer reservoir will not flow back through this fluid channel towards the mixing unit.

The fluid channel between the venting hole and the buffer reservoir may output in the buffer reservoir at a position above the liquid-air interface when the cartridge is in the second orientation. Hence, when the cartridge is arranged flat on a table, i.e. corresponding to the second orientation, liquid in the buffer reservoir can be prevented from flowing into the fluid channel towards the venting hole.

The buffer reservoir can be vertically elongated when the cartridge is held in an orientation in which a normal to the fluid channel plane is horizontal, e.g. the first orientation. In this manner, liquid that is outputted by the mixing unit into the buffer reservoir only sees a relatively small area in which it may be exposed to the gas(ses) in the buffer reservoir. By having the elongated shape, the stability of microbubbles can therefore be improved.

The buffer reservoir can be transparent to allow a user to visually inspect its contents or the level thereof.

It should be noted that the abovementioned features related to the buffer reservoir would equally be advantageous for applications other than the formation of microbubbles.

The cartridge may further comprise filters arranged in each of said one or more gas inlets formed in the cartridge body, and, when applicable, in the venting hole. These filters are configured to prevent bacteria and/or other pathogens from entering the cartridge. These filters are preferably hydrophobic. At least one filter may comprise a filter membrane, a first filter support, and a second filter support, wherein the first and second filter supports are fixedly attached to or integrally formed with the cartridge body and wherein the filter membrane is arranged in between the first and second filter supports. The first and second filter supports prevent that the filter membranes are removed from the cartridge or are ruptured of torn in case of sudden changes in under-pressure or over-pressure. The first and second filter supports may comprise ribs extending over the openings for the gas inlet(s) and venting hole either from an outside of the cartridge body or from an inside of the cartridge body.

The cartridge may have been formed by fixedly attaching a first cartridge part and a second cartridge part using ultrasonic welding, each cartridge part comprising a base layer. Prior to fixedly attaching these parts, one of the first and second cartridge part may have comprised ridges and/or protruding portions extending from the base layer that were configured to cooperate with protruding portions and/or ridges extending from the base layer of the other of the first and second cartridge part during ultrasonic welding. As a result of the ultrasonic welding, the protruding portions have become integrally connected to the corresponding ridges. The integrally connected ridges and protruding portions, and the base layer of the first and second cartridge parts may together define at least one of the mixing unit, the fluid storage system, the one or more gas inlets, the outlet, the buffer reservoir, and the fluid channels for connecting them. Preferably, all of these structures are formed in this manner. The first and second cartridge parts can be made, using injection molding, from one or more materials from the group of thermoplastic materials such as polycarbonate.

According to a second aspect, the present invention provides a cartridge system that comprises a cartridge as described above and a device comprising a housing with an opening in which the cartridge can be releasably inserted. The device comprises one or more nozzles for inserting a respective pressurized gas into the one or more gas inlets, respectively, for the purpose of said mixing by the mixing unit of the cartridge.

The cartridge can be configured as comprising the abovementioned sealed off container. In this case, the device may further comprise an engaging unit for engaging the sealed off container arranged in a storage chamber of at least one fluid storage unit for the purpose of causing the sealed off container to break, rupture, or to become cut or pierced.

The device may comprise a drive system for bringing the nozzles and/or engaging unit into and out of engagement with the one or more gas inlets and the sealed off container, respectively. The device may comprise a controller for controlling the drive system. Moreover, at least one of said one or more nozzles may be connected to a controllable valve, wherein the controller is configured for controlling a flow of pressurized gas through said at least one of said one or more nozzles via the controllable valve. The device may comprise one or more reservoirs for holding pressurized gas, said one or more reservoirs being connected to the one or more nozzles. Alternatively, the device may comprise one or more further gas inlets that are connected to the one or more nozzles, respectively, wherein the one or more further gas inlets are configured to be connected to one or more gas reservoirs external to the device. Accordingly, the device can rely on internal storage of the required gases or may be connected to an outside infrastructure by which the required gases are provided. **In** both cases, the controllable valves are preferably arranged in the device.

The controller may be configured to control the device to be operable in a first state in which the nozzles and engagement unit are positioned at a distance from the cartridge. The system may further be operable in a second state in which the controller controls the drive system to bring the nozzles into contact with the one or more gas inlets and wherein the controller controls the controllable valve(s) such that pressurized gas(ses) is/are fed to the cartridge via the respective nozzle(s) and gas inlet(s) of the cartridge. **In** this manner, the various fluid channels inside the cartridge and optionally the buffer reservoir can be flushed with one or more prescribed gases.

The device may further be operable in a third state in which the controller controls the drive system to cause the engaging unit to engage the sealed off container for the purpose of causing the sealed off container to break, rupture or to become cut or pierced, and to subsequently control the controllable valve(s) to provide pressurized gas(ses) to the cartridge via the respective nozzle(s) and gas inlet(s) of the cartridge for the purpose of said mixing by the mixing unit of the cartridge. Typically, some time is reserved between the moment of applying the propellant gases and the moment at which the sealed off container releases its liquid to allow all or most of the liquid to be collected in the liquid reservoir of the relevant fluid storage unit prior to the mixing process.

The controller may be configured to control the controllable valve(s) to stop the supply of pressurized gas(ses) to the cartridge when changing from the second state to the third state. **In** this manner, the flow of the liquid from the sealed off container to the liquid reservoir is not affected.

The drive system may comprise a first unit in which the one or more nozzles are moveably mounted and in which the engagement unit is mounted. The drive system may further comprise an actuator for moving the first unit relative to the cartridge when the cartridge is inserted into the device.

The nozzles can be mounted in a spring-biased manner in the first unit to allow the nozzles to move relative to the first unit in a first direction towards the cartridge and perpendicular thereto. As described above, the first unit may move towards the cartridge to allow the nozzles to engage the cartridge. At that time, the engagement unit is not engaging the sealed off container. Such engagement is obtained when the first unit moves even further towards the cartridge. By having the nozzle(s) mounted in the first unit in a spring biased manner, the nozzle(s) may move back towards the first unit during this last movement of the first unit while maintaining engagement with the gas inlet(s). In this manner, damage of the nozzle(s) and/or gas inlet(s) of the cartridge can be prevented.

The engagement unit may be movably mounted to the first unit. Movement of the engagement unit relative to the first unit can be used to accommodate for non-zero tolerances in the relative position and/or shape of the sealed off container and the gas inlet(s). For example, the engagement unit may be able to translate in one or more directions other than the direction towards the cartridge, and/or to rotate about these directions. Similar degrees of freedom may apply to one or more nozzles. In both cases, the degrees of freedom are used to accommodate for tolerances in the manufacturing process of the cartridge. Put differently, the degrees of freedom allow alignment of the nozzles and engagement unit relative to the cartridge.

The drive system may further comprise a second unit that is coupled to the first unit, wherein the first unit is able to move in at least one degree of freedom relative to the second unit, and wherein the actuator is configured to move the second unit in the first direction, i.e. towards the cartridge. In this manner, the movement of the second unit is completely dictated by the actuator. However, movement of the first unit may generally also depend on the alignment of the nozzles and engagement unit with the cartridge. This is made possible by the fact that the first unit may move relative to the second unit. The mutual movement between the first and second units can be made possible by having the second unit coupled to the first unit using one or more bended leaf springs. Hence, the actuator will drive the first unit indirectly, i.e. via the second unit.

The first unit may be able to move relative to the second unit in a second direction orthogonal to the first direction and in a third direction orthogonal to both the first and second direction. Referring to the first direction towards the cartridge as the z-direction, the first and second direction may correspond to an x-direction and a y-direction. respectively. The first unit can further be able to rotate about the first direction relative to the second unit. Preferably, the first unit is only able to move relative to the second unit by translating in the x-direction and/or y-direction and by rotating about the z-direction.

A nozzle among the one or more nozzles can be able move along the first direction and to rotate about the first direction relative to the first unit, and wherein another nozzle among the one or more nozzles is able to move along the first direction, the second direction, and the third direction, and to rotate about one of the second and third direction. For example, in case two nozzles are used, a first nozzle is only able to move along the z-direction and to rotate about this direction. The other nozzle may in this case be able to move only along the x-direction, the y-direction, and the z-direction, and to rotate about the y-direction. The other degrees of freedom for these nozzles may be fixed.

The drive system may comprise a threaded spindle that is driven by the actuator and which extends in the first direction and wherein the second unit is coupled to the threaded spindle to cause a translation of the second unit in the first direction when the threaded spindle rotates. Preferably, rotation of the spindle will only cause a movement of the second unit in the first direction.

The device may further comprise a second frame that is fixedly connected to the housing, wherein the threaded spindle is rotatably received in the second frame, the second frame preferably comprising a wall segment that extends substantially perpendicular to the first direction and in which the threaded spindle is rotatably received.

The device may comprise a force sensor for sensing a force with which the engagement unit is pressing against the sealed off container, wherein the controller is configured to control the drive system in dependence of the sensed force.

The abovementioned second frame may comprise a first part and a second part coupled to the first part, wherein the first and second parts are able to move relative to each other when a force is exerted onto the cartridge by the engagement unit. In this case, the force sensor can be configured for determining said force in dependence of a mutual displacement between the first and second parts. For example, the first and second parts may be connected using a structure that is compressible in the first direction, such as a meandering bar or a lattice of bars. The compression can be determined using a position sensor in a known manner. The output of the position sensor in combination with known mechanical properties of the compressible structure can then be used to calculate the force exerted onto the cartridge or a parameter that represents and/or corresponds to such force.

According to a third aspect, the present invention provides a cartridge configured for mixing a liquid held in the cartridge, within the cartridge, with a further liquid held in the cartridge or with a pressurized gas supplied to the cartridge, wherein the liquid is intended for intracorporeal use in a human or animal, for example for intravenous use or intra-cavity use, wherein the liquid held in the cartridge comprises or is used to form a diagnostic agent, such as a contrast agent for medical imaging, or a therapeutic agent, such as a medicament. This cartridge may be configured as the cartridge defined before.

According to a fourth aspect, the present invention provides a cartridge system, comprising the cartridge in accordance with the third aspect of the invention and a device in which the cartridge can be releasably inserted, wherein the device is configured for providing pressurized gas(ses) to be used as propellant(s) for moving the liquid(s) inside the cartridge for the purpose of mixing the liquid(s), and/or to be used as gas to be mixed. This device can be configured as described before.

Next, the invention will be described in more detail referring to the appended drawings, wherein:
Figure 1 illustrates a perspective view of an embodiment of a cartridge system in accordance with the present invention;
Figures 2 and 3 present different views of the cartridge of the cartridge system of figure 1;
Figure 4 illustrates details regarding the construction of the cartridge of figure 2;
Figure 5 illustrates a microfluidics device used in the cartridge of figure 2;
Figure 6 illustrates the cooperation between a sealed off container and the cartridge of figure 2;
Figure 7 illustrates details regarding the filters used in the cartridge of figure 2;
Figure 8 illustrates the inside of the device shown in figure 1;
Figures 9-14 illustrate different detailed views of different parts of the inside of the device shown in figure 8; and
Figure 15 schematically illustrates the cartridge system of figure 1.

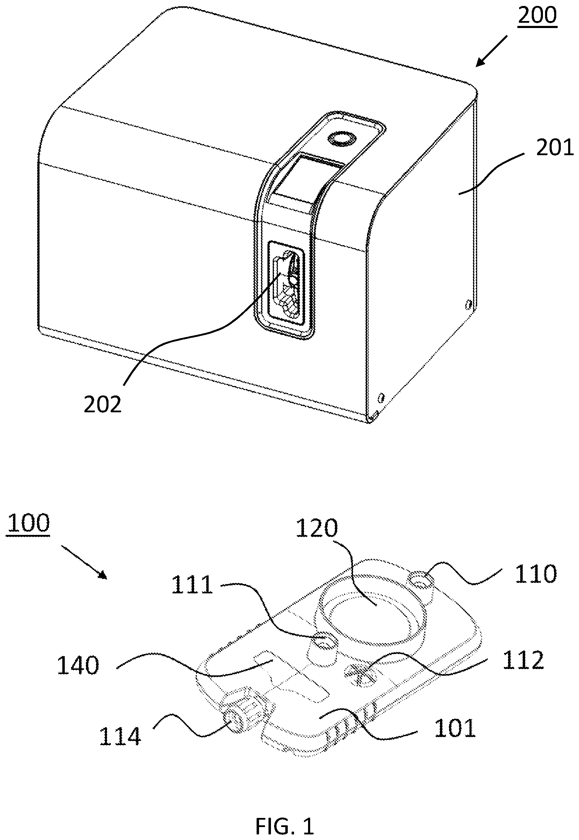

Figure 1 illustrates an embodiment of a cartridge system in accordance with the present invention. The system comprises a cartridge 100 and a device 200. The latter device comprises a housing 201 and an opening 202 in housing 201 in which cartridge 100 can be releasably inserted. In fact, device 200 is shown having a cartridge 100 inserted therein.

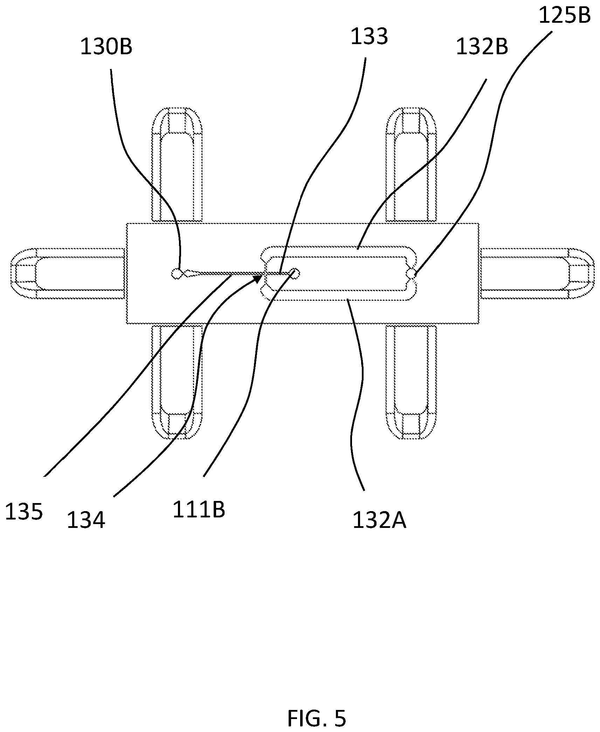

Cartridge 100 comprises a cartridge body 101 and is configured for mixing a liquid held in a sealed off container 120, within the cartridge, with a gas supplied through gas inlet 111. To that end, cartridge 100 comprises a mixing unit in the form of a microfluidics device that generates microbubbles filled with the gas that is supplied via gas inlet 111. This mixing unit will be discussed in more detail later in connection with figure 5.

As the microfluidics device is a passive device, i.e. it requires energy to work, a pressurized gas has to be fed to gas inlet 111 and towards the microfluidics device. Moreover, once the liquid is released from container 120, a pressurized gas will be fed to gas inlet 110 to push the released liquid towards the microfluidics device. Put differently, the gas supplied to gas inlet 110 will be used as a propellant for propelling the liquid released from container 120 towards the microfluidics device.

The liquid in container 120 can be released by breaking container 120. To that end, sufficient force should be exerted onto container 120 as will be explained later.

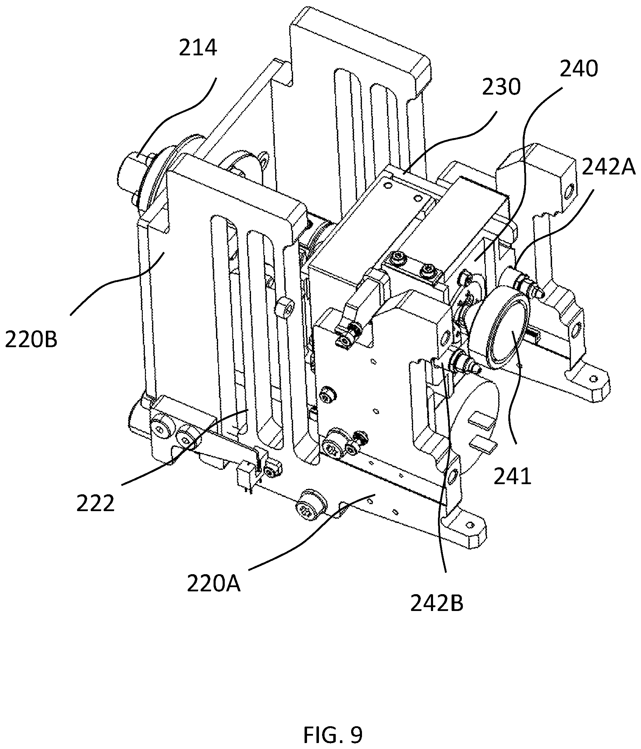

Device 200 is configured to perform the actions described above. In other words, when a cartridge 100 is inserted in opening 202, device 200 will ensure that appropriate gases are supplied to cartridge 100 and that container 120 will be broken. This functionality will be explained later when referring to figures 9-15.

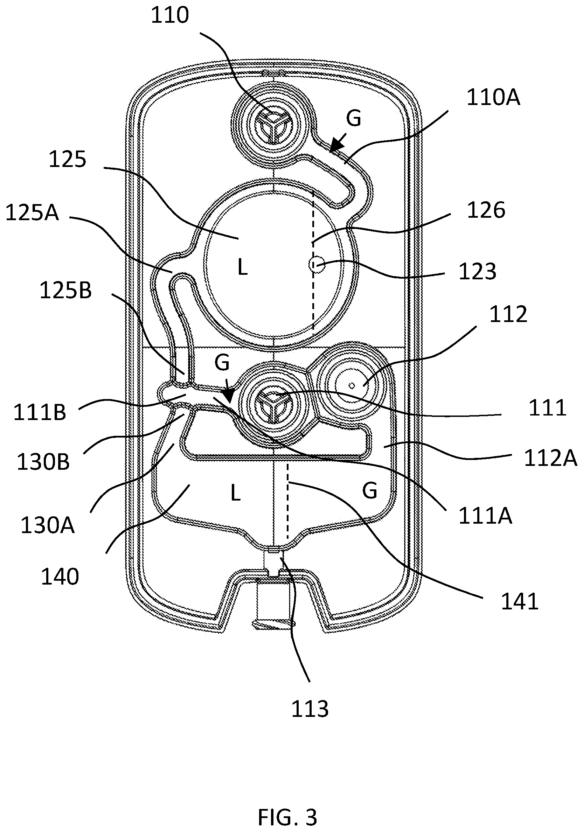

An output of the microfluidics device is connected to a buffer reservoir 140 in which the mixed liquid can be temporarily stored. This reservoir, which will be discussed in more detail in connection with figure 3, is connected to a venting hole 112 to prevent excessive pressure build up in reservoir 140. Furthermore, buffer reservoir 140 is connected to an output 113 of cartridge 100, which in figure 1 is covered by a closing cap 114.

As shown in figure 1, sealed off container 120 comprises a blister package. This package is configured such that when a force is exerted on the topside of the package in figure 1, the backside of the package will bend towards cartridge body 101. Container 120 may comprise a solution of phosphate-buffered saline (PBS) to which phospholipids have been added. An exemplary volume of liquid held in container 120 is about 2 ml.

The gas supplied to gas inlet 110, which will be used as propellant, may be SF₆. This same gas may be fed to gas inlet 111. As will be explained later, based on these fluids, the microfluidics device will generate a suspension of microbubbles filed with SF₆ in an aqueous solution. Such suspension may be used as a contrast agent for ultrasound imaging.

The size of the SF₆ core of the microbubble in combination with the shell formed by the phospholipids determines the resonance frequency of the microbubble. When the microbubble is subjected to an ultrasound wave of a suitable frequency, equaling or at least approaching the resonance frequency of the microbubble, the bubble will resonate at the resonance frequency of the microbubble. This resonance can be picked up by the ultrasound imaging apparatus. In this manner, a high contrast can be achieved between microbubble-rich and microbubble-poor regions.

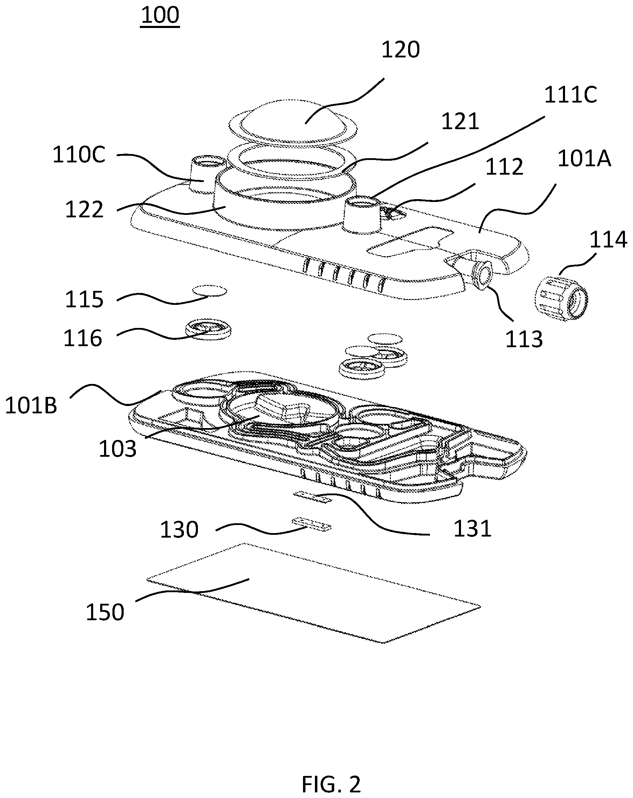

Figure 2 illustrates an exploded view of cartridge 100. Here, an adhesive 121 can be seen with which container 120 is fixedly attached to cartridge body 101. Furthermore, cartridge body 101 comprises a first part 101A and a second part 101B that are fixedly connected to each other using ultrasonic welding or some other form of monolithic bonding.

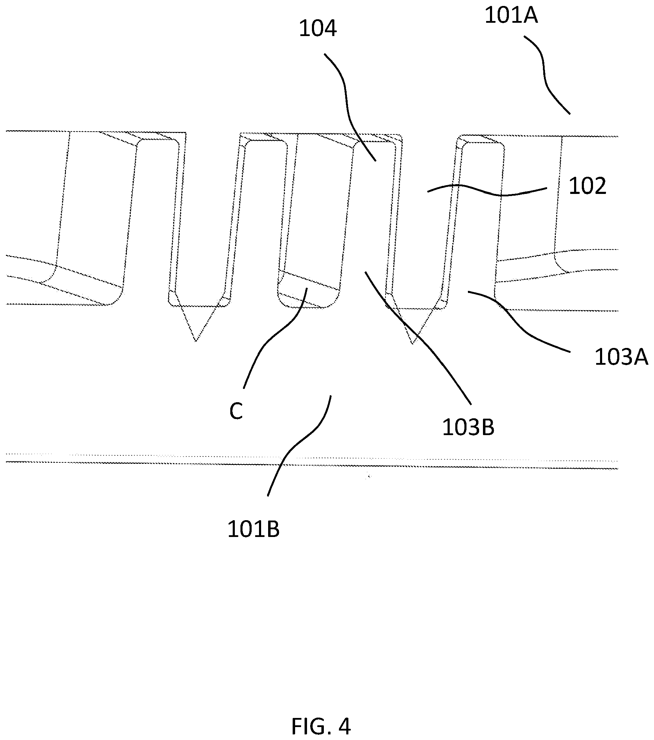

Both first part 101A and second part 101B comprise ridges and/or protrusions that extend from a base layer. This base layer may comprise structures, such as recesses, for the formation of structures in the final cartridge such as buffer reservoir 140. Examples of protrusions 102 and ridges 103 are shown in figure 4. Here, each ridge 103 comprises a pair of ridge parts 103A, 103B in between which an opening is formed. In this opening, a protrusion 102 of the other body part may extend once the first part 101A and second part 101B are properly mutually aligned. Thereafter, parts 101A and 101B are mutually connected using ultrasonic welding as a result of which the tip of protrusion 102 will be melted to make an integral connection to ridge parts 103A and 103B. In figure 4, two ridges are connected in the manner described above. Consequently, a fluid channel, indicated by rectangle "C", is created in between two ridges.

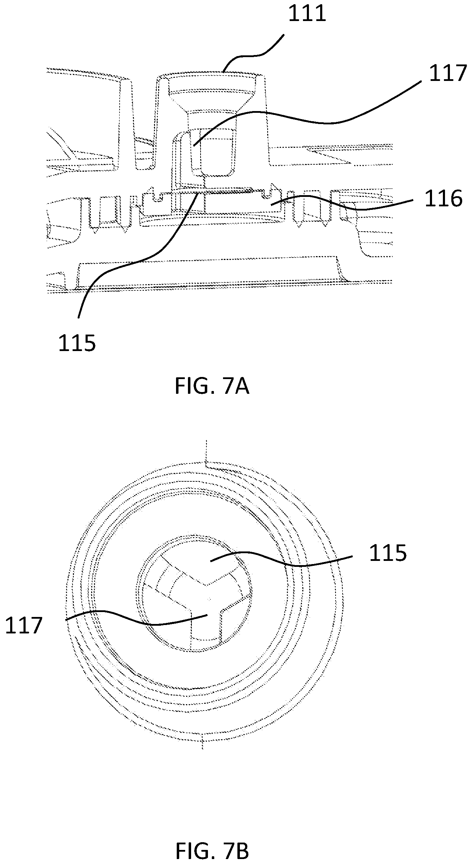

Now returning to figure 2, gas inlets 110 and 111 are each formed using a cylindrical protrusion 110C and 111C, respectively. The protrusion extends from body part 101A. Moreover, in between body parts 101A, 101B, hydrophobic filter membranes 115 are arranged at the position of inlets 110, 111 and venting hole 112. These filters are supported on two sides thereof by suitable support structures. Figure 2 illustrates a first filter support 116 that is connected to body part 101A using ultrasonic welding before attaching body parts 101A, 101B. On the other side of filter membrane 115 a second filter support 117 is provided that is star-shaped, see figures 7A and 7B for a cross-sectional view and a top view of gas inlet 111, respectively.

Again returning to figure 2, a microfluidics device 130 can be mounted to cartridge body 101, and more in particular to body part 101B, using a suitable adhesive which, in figure 2, is represented as component 131.

Figure 2 further illustrates that a cover 150 may be mounted on the backside of cartridge 100 thereby covering microfluidics device 130. On this cover 150, data about cartridge 100 may be printed.

Figure 3 illustrates a schematic top view of cartridge 100. In this view, the various components of cartridge 100 are shown in more detail. It should be noted that in this figure various gas-liquid interfaces 126, 141 are indicated to indicate the boundaries between liquid media (L) and gaseous media (G). Interfaces 126, 141 reveal that the configuration shown in figure 3 corresponds to cartridge 100 still being inside device 200. In other words, Earth's gravity works in the direction from right to left in figure 3.

As can be seen in figure 3, from gas inlet 110 a fluid channel 110A extends to a liquid reservoir 125. In this reservoir, liquid from container 120 will be collected as will be explained later. At a bottom part of reservoir 125, a fluid channel 125A extends towards an opening 125B in cartridge body 101. From gas inlet 111, a fluid channel 111A extends to an opening 111B in cartridge body 101.

Openings 125B, 111B are used for transporting liquid and gas, respectively, to microfluidics device 130, which is illustrated in figure 5. In this figure, it can be seen that opening 125B is connected to a first channel 132A and a second channel 132B in microfluidics device 130, and that opening 111B is connected to a relatively short channel 133 in microfluidics device 130.

First and second channels 132A, 132B and channel 133 exit in a flow focusing junction 134. At this junction, a flow of the SF₆ gas inside channel 133 is restricted by flows of the liquid in channels 132A, 132B such that SF₆ filled microbubbles are generated in bubble formation channel 135. The liquid having the microbubbles is outputted from microfluidics device 130 through opening 130B in cartridge body 101. It is noted that the operation of microfluidics device 130 is known from WO 2013/141695 and WO 2016/118010. Microfluidics device 130 is typically fabricated from a glass substrate, such as borosilicate glass, or a polymeric material.

Now referring again to figure 3, the liquid outputted by microfluidics device 130 is fed via opening 130B and fluid channel 130A to a buffer reservoir 140. This reservoir is elongated in the vertical direction. Consequently, when liquid enters buffer reservoir 140, it will be less likely to mix with gaseous media in reservoir 140 than if buffer reservoir 140 were elongated in the horizontal direction.

Via fluid channel 112A, which extends between buffer reservoir 140 and venting hole 112, excess pressure can be relieved.

Buffer reservoir 140 is connected to outlet 113. Buffer reservoir 140 can be extracted through outlet 113 using a syringe. A Luer taper connection is used for outlet 113 to provide a sealed connection between the syringe and outlet 113. It is noted that when liquid is extracted from buffer reservoir 140, ambient air may be attracted through venting hole 112 for pressure equalization thereby preventing severe under-pressure in buffer reservoir 140 that would destroy the microbubbles and complicate the extraction of the liquid.

After the liquid is collected in buffer reservoir 140, cartridge 100 can be taken out of device 200. Typically, the user will then place cartridge 100 on a supporting surface, such as a table. To prevent the inadvertent flow of liquid from reservoir 140 into fluid channel 130A, it is ensured that in this orientation of cartridge 100, fluid channel 130A and fluid channel 112A exit into buffer reservoir 140 at a position above the gas-liquid interface. Accordingly, to achieve this advantage, the capacity of reservoir 140 should be chosen in dependence of the volume of liquid in container 120.

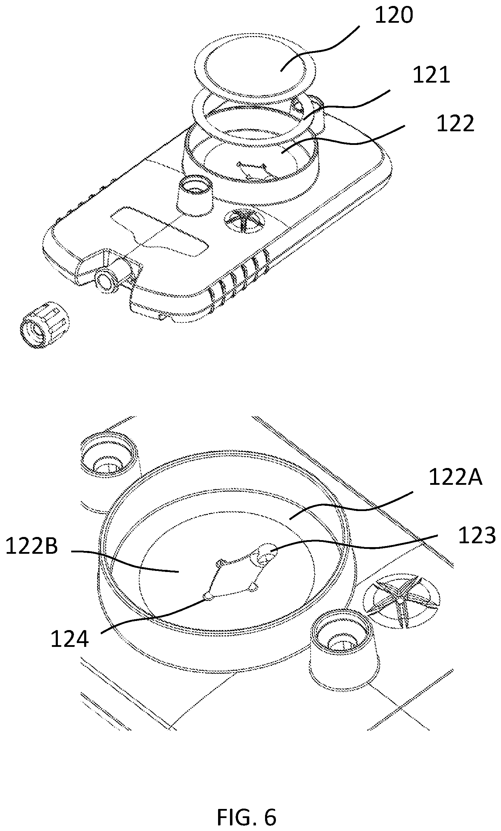

Now referring to figure 6, container 120 is arranged in a storage chamber 122 that has a support surface comprising an edge portion 122A and a center portion 122B. Container 120 can be fixedly attached to edge portion 122A using a suitable adhesive, indicated in figure 6 as component 121.

From center portion 122B, three needles 124 extend toward container 120. When sufficient force is exerted on the topside of container 120, the backside thereof will bend downward thereby engaging needles 124. Consequently, the backside of container 120 will be punctured and the liquid inside container 120 will be released. This liquid will flow through an opening 123, which is located at an upper portion of center portion 122B. Once flown through opening 123, the liquid will be collected in liquid reservoir 125. Consequently, when cartridge 100 is inside device 100, a small amount of liquid will remain in storage chamber 122 below opening 123. The positioning of opening 123 should be such that when cartridge 100 is inside device 200, opening 123 remains above gas-liquid interface 126. In this manner, the gas fed through gas inlet 110 cannot push liquid inside liquid reservoir back into storage chamber 122.

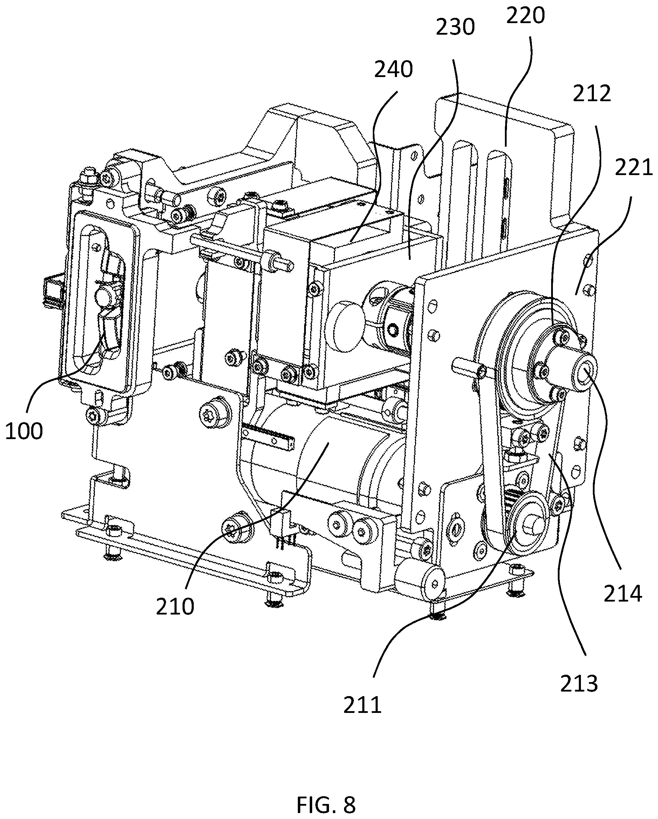

Next, the functionality of device 200 will be described referring to figures 8-15. First, in figure 8, the most relevant components of device 200 are shown. For example, device 200 comprises an actuator 210, e.g. an electromotor, that drives a threaded spindle 214 via a first gear 211 and a second gear 212 that are coupled using a belt 213.

Spindle 214 is rotatably mounted in wall segment 221. This wall segment is part of a frame 220 that is fixedly connected to housing 201. Here, it is noted that figure 8 only illustrates part of frame 220 for illustrative purposes.

As shown in figure 9, frame 220 comprises a first part 220A and a second part 220B that are connected using a number of parallel and spaced part bars 222. The provision of bars 222 allows first part 220A to move towards second part 220B and vice versa. As will be explained later, this feature will be used for creating a force sensor.

Figure 9 further illustrates a nozzle 242A for inserting pressurized SF₆ gas through gas inlet 110 and a nozzle 242B for inserting pressurized SF₆ gas through inlet 111. It is noted that different inlets 110, 111 are used despite the fact that they carry the same gas, because the functionality of this gas is different, i.e. use as a mixing gas or use a propellant. Figure 9 further illustrates an engagement unit 241 that is used to exert force onto reservoir 120. Nozzles 242A, 242B, and engagement unit 241 are each moveably mounted in unit 240.

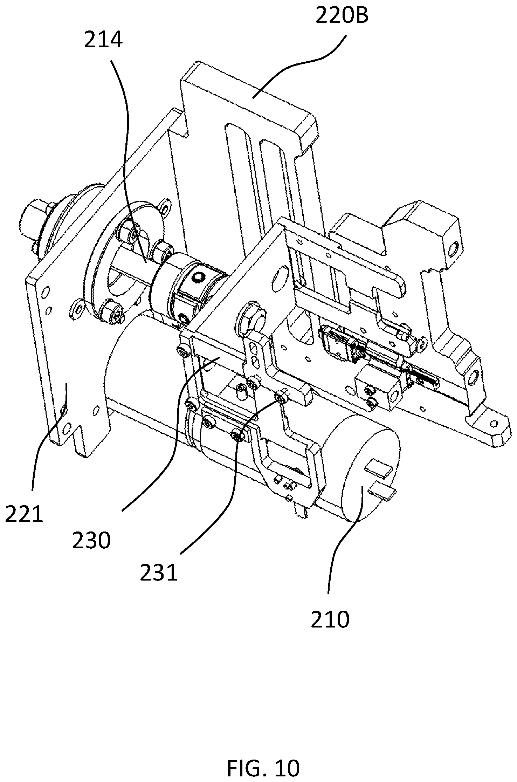

Now referring to figure 10, threaded spindle 214 is connected to a U-shaped frame 230. More in particular, when spindle 214 rotates, frame 230 will only move in the direction towards or from cartridge 100. This direction will hereinafter be referred to as the z-direction. In addition, the x-direction will correspond to the direction orthogonal to the z-direction and corresponding to the direction along which cartridge 100 is inserted into device 200. The remaining y-direction is orthogonal to the z-direction and the x-direction.

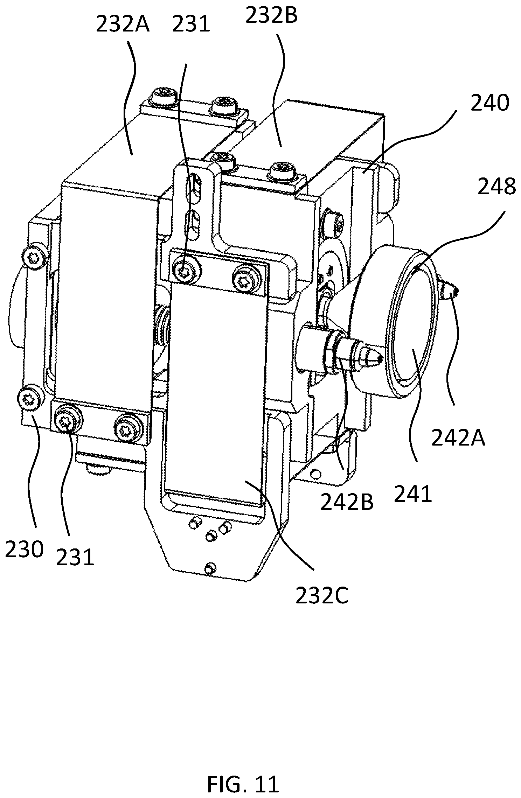

Figure 11 illustrates various folded leaf springs 232A-232C that connect U-frame 230 to unit 240 using screws 231. Springs 232A-232C are configured such that unit 240 is able to move relative to U-frame 230. More in particular, unit 240 is only able to move in the x-direction, the y-direction, and to rotate about the z-direction relative to U-frame 230.

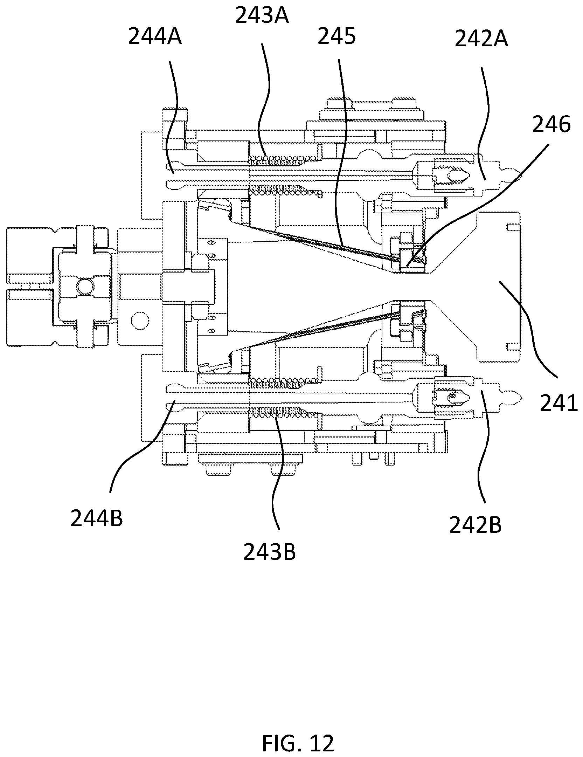

Figure 12 illustrates a cross section of unit 240. This figure illustrates that nozzles 242A, 242B are mounted in a spring-biased manner in unit 240 using springs 243A, 243B, respectively. Gas is fed to nozzles 242A, 242B through nozzle inlets 244A, 244B, respectively. Typically, these inlets are connected to tubing (not shown).

In figure 12, nozzle 242A is able to move in the z-direction and may rotate about this direction relative to unit 240. Nozzle 242B is mounted in a slotted hole allowing nozzle 242B to move in the x-direction and y-direction and to rotate about the y-direction relative to unit 240.

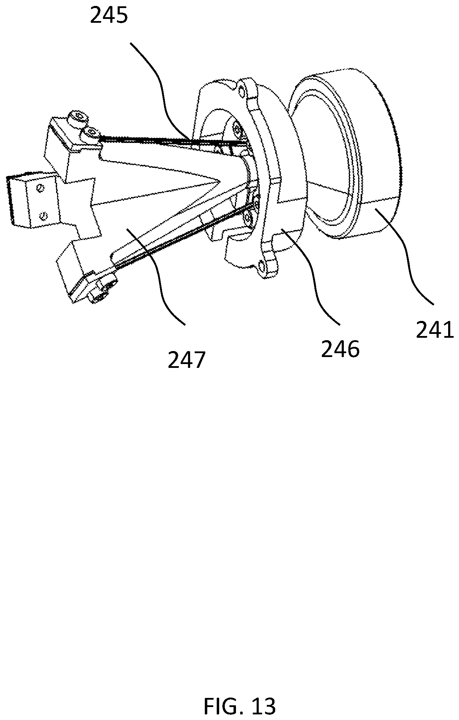

Now referring to figure 13, engagement unit 241 is fixedly connected to a tripod like structure 247. Engagement unit 241 is arranged through a connecting ring 246. This connecting ring is connected through rods 245 to the bottom of structure 247.

Now referring to both figures 12 and 13, connecting ring 246 is mounted in a fixed manner in unit 240. However, the provision of rods 245 allows engagement unit 241 to rotate about the x-direction and y-direction relative to unit 240. In this manner, engagement unit 241 can adjust its position relative to cartridge 100 to optimally engage container 120. Furthermore, to avoid leakage, an O-ring (not shown) can be placed in a groove 248 on the contact area of engagement unit 241.

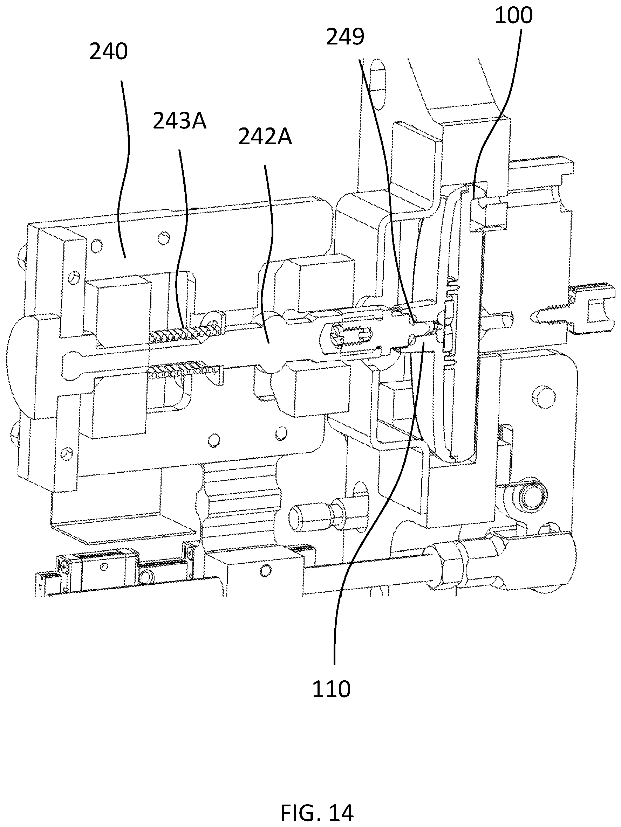

Figure 14 presents a partial cross-section illustrating how nozzle 242A engages gas inlet 110 of cartridge 100. Nozzle 242A can be provided with an O-ring 249 to provide a sealed connection with gas inlet 110.

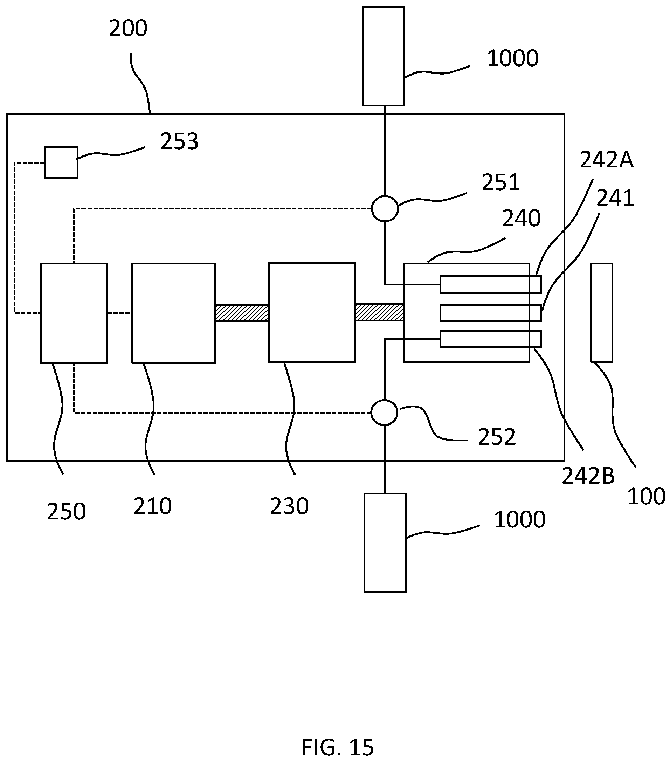

Next, a possible operational cycle of the cartridge system of figure 1 will be described referring to the schematic illustration of this system in figure 15.

As a first step, cartridge 100 is mounted in device 200 through opening 202 in figure 1. Thereafter, a controller 250 in device 200 may optionally perform various checks, such as a gas leakage test or a check that cartridge 100 is properly positioned.

When cartridge 100 is just placed in opening 202, the system and therefore device 200 is in a first state in which unit 240 and U-frame 230 are positioned relatively far away from cartridge 100. Thereafter, controller 250 will operate actuator 210 to move U-frame 230 towards cartridge 100. As a result, nozzles 242A, 242B will engage inlets 110, 111. Due to the degrees of freedom of these nozzles and the degrees of freedom of unit 240 relative to U-frame 230, an alignment of unit 240 relative to cartridge 100 will be obtained. In addition, when properly engaged, controller 250 will control controllable valves 251, 252 that are arranged in between a container 1000 with pressurized SF₆ and nozzles 242A, 242B. More in particular, valves 251, 252 are controlled to allow gas inlets 110, 111 and the components of cartridge 100 connected thereto to be flushed with SF₆. This is referred to as the second state of the system and device 200. When the flushing action is finished, controller controls actuator 210 to move U-frame 230 and therefore unit 240 closer to cartridge 100 such that engagement unit 241 can engage container 120. During this movement, nozzles 242A, 242B will move backwards relative to unit 240 against the spring-biasing force. In fact, the spring constant of springs 243A, 243B determines to a large extent the force with which nozzles 242A, 242B are pressing on inlets 110, 111, respectively. It should further be noted that a single container 1000 may also be used as the gas supplied to inlets 110, 111 is identical.

During the movement of unit 240, controller 250 checks the force exerted by engagement unit 241 onto reservoir 120 using force sensor 253. This latter sensor may already have sensed a force caused by the nozzles 242A, 242B engaging inlets 110, 111, respectively. In particular, the reaction force exerted onto nozzles 242A, 242B by inlets 110, 111, respectively, is transferred via unit 240, U-frame 230, and spindle 214 onto wall segment 221. Consequently, as can be derived from figure 9, frame part 220B tends to move away from frame part 220A. This displacement is made possible by bars 222 and is measured by a position sensor. Force sensor 253 calculates a resulting force based on the observed displacement. It should be noted that force sensor 253 may be integrated in controller 250. In addition, the actual force need not be calculated as a parameter representing this force may also be sufficient.

When engagement unit 241 presses sufficiently hard on reservoir 120 the latter will break and will release its liquid to liquid reservoir 125. Thereafter, valves 251, 252, which were typically closed during the time the liquid was released from container 120, are controlled to start the mixing process. Valves 251, 252 are preferably controlled after the liquid has reached the liquid reservoir 125.

During the mixing process, SF₆ is fed to inlet 110 to act as propellant to move the liquid out of liquid reservoir 125 to microfluidics device 130. At the same time, SF₆ is fed to inlet 111 as the gas to be used by microfluidics device 130 for generating microbubbles.

After a predetermined amount of time, valves 251, 252 are closed by controller 250 and unit 240 is moved away from cartridge 100. Thereafter, cartridge 100 can be removed from device 200 with the microbubble suspension being held in buffer reservoir 140.

The last part of the process, i.e. moving unit 240 towards cartridge 100 thereby allowing engagement unit 241 to engage reservoir 120 and the subsequent mixing process is performed when the system and therefore device 200 is in a third state.

In the above, the present invention has been explained using an embodiment that was targeted at the creation of a microbubble suspension intended to be used intracorporeally as a contrast agent. The skilled person will however appreciate that the present invention is not limited to such application. Other applications wherein a sterile liquid held in the cartridge is to be mixed, within the cartridge, with another sterile liquid held in the cartridge or a gas supplied to the cartridge, are equally possible.

Using the system of the application, a user may repeatedly generate case or person specific mixing products that can be generated just before use. Sterility of the end product is guaranteed because the crucial components, i.e. the liquid(s), are held in the container and because the mixing process also occurs in the cartridge. Contact with external devices, i.e. device 200, only involves the exchange of gaseous media. This exchange can be performed in a sterile manner using off-the-shelf filters.

Accordingly, an advantage of the present invention is that device 200 can be arranged in a non-sterile environment, for example an examining room in a hospital, while still allowing a sterile mixing product to be obtained.

In view of the above, it must be concluded that the present invention is not limited to the embodiments shown. Instead, various modifications are possible and other applications can be realized without departing from the scope of the invention, which is defined by the appended claims.

## Claims

1. A cartridge (100), comprising:
a cartridge body (101);
one or more gas inlets (110, 111) formed in the cartridge body (101) configured for receiving one or more pressurized gases, respectively;
a fluid storage system formed in the cartridge body (101) and comprising a fluid storage unit that includes a fluid storage unit inlet that is connected to a first gas inlet among the one or more gas inlets (110, 111) and a fluid storage unit outlet, wherein the fluid storage unit is configured for holding a liquid and for outputting said liquid through its fluid storage unit outlet in response to a first pressurized gas among the one or more pressurized gases being supplied to it through said first gas inlet by using the first pressurized gas as a propellant; and
a mixing unit arranged in or mounted to the cartridge body (101) and being in fluid communication with the fluid storage unit using a fluid channel (125A) formed in the cartridge body (101), said mixing unit being configured for mixing the liquid outputted from the fluid storage unit with a second pressurized gas among the one or more pressurized gases, wherein:
in the case that the first pressurized gas is the same gas as the second pressurized gas, the second pressurized gas is received through the first gas inlet or through a second gas inlet among the one or more gas inlets (110, 111), and
in the case that the first pressurized gas is different from the second pressurized gas, the second pressurized gas is received through a second gas inlet among the one or more gas inlets (110, 111);
**characterized in that** the fluid storage unit comprises:
a storage chamber (122) configured for receiving a sealed off container (120) in which the liquid is held in a sterile and sealed off manner; and
a liquid reservoir (125) in fluid communication with the storage chamber (122), wherein the fluid storage unit inlet is connected to one of the storage chamber (122) and the liquid reservoir (125) and wherein the fluid storage unit outlet is connected to the liquid reservoir (125);
wherein the liquid reservoir (125) is configured for collecting liquid that is released from the sealed off container (120) after the sealed off container (120) has been broken, ruptured, cut, or pierced.

2. The cartridge (100) according to claim 1, wherein the liquid held in the fluid storage system is intended for intracorporeal use in a human or animal, for example for intravenous use or intra-cavity use, wherein the liquid preferably comprises or is used to form a diagnostic agent, such as a contrast agent for medical imaging, or a therapeutic agent, such as a medicament.

3. The cartridge (100) according to any of the previous claims, wherein the sealed off container (120) comprises a blister package and/or wherein the sealed off container (120) is fixedly held in the storage chamber (122), for example by means of an adhesive.

4. The cartridge (100) according to any of the previous claims, wherein the liquid reservoir (125) is formed as a part of the storage chamber (122), or wherein said fluid storage unit comprises a fluid channel formed in the cartridge body (101) connecting the storage chamber (122) and the liquid reservoir (125).

5. The cartridge (100) according to any of the previous claims, wherein the storage chamber (122) comprises a supporting surface for supporting the sealed off container (120) and at least one protruding pin or needle extending towards the sealed off container (120) for the purpose of puncturing through the sealed off container if sufficient force is exerted onto the sealed off container (120), wherein the fluid storage unit is preferably provided with a protective ring that protrudes away from the cartridge body (101) further than the sealed off container (120) when it is placed in the storage chamber (122).

6. The cartridge (100) according to any of the previous claims, wherein the mixing unit is configured to mix the liquid received from the fluid storage system with the second pressurized gas, said mixing unit comprising a microfluidics device (130) configured for generating microbubbles within said liquid that are filled with the second pressurized gas.

7. The cartridge (100) according to claim 6, wherein the microfluidics device (130) is configured for generating microbubbles having a diameter below 10 micrometer, and preferably in a range of 2-5 micrometer; and/or
wherein the second pressurized gas comprises a gas from the group consisting of SF₆, N₂, CO₂, O₂, H₂, He, Ar, ambient air, and perfluorocarbon gases, such as CF₄, C₂F₆, C₂F₈, C₃F₆, C₃F₈, C₄F₆, C₄F₈, C₅F₁₀, C₅F₁₀, C₅F₁₂ and mixtures thereof; and/or
wherein said liquid received from the fluid storage system comprises a liquid from the group consisting of water, dispersion of lipids, such as phospholipids, or proteins in an aqueous solution, active pharmaceutical ingredients, and alcohols.

8. The cartridge (100) according to any of the claims 6-7, wherein the microfluidics device (130) comprises:
a first inlet (111B) for receiving the second pressurized gas;
a second inlet (125B) for receiving said liquid;
a bubble formation channel (135) for generating the microbubbles based on a flow of the second pressurized gas received through the first inlet and a flow of the liquid received through the second inlet;
wherein, preferably, the cartridge body (101) comprises a first opening, a second opening, and a third opening, wherein the first opening is in fluid communication with the gas inlet among the one or more gas inlets that receives the second pressurized gas, and wherein the second opening is in fluid communication with the fluid storage system for the purpose of receiving the liquid, wherein the microfluidics device (130) is positioned relative to the cartridge body (101) such that the first opening is aligned with the first inlet of the microfluidics device (130), the second opening with the second inlet of the microfluidics device (130), and the third opening with an outlet of the microfluidics device (130), wherein the microfluidics device (130) is fixedly connected to the cartridge body (101) using an adhesive or a monolithic bond, or wherein the microfluidics device (130) is integrally formed with the cartridge body (101);
wherein the microfluidics device (130) preferably comprises:
a flow focusing junction (134);
a first channel (132A) connected on one end to the second inlet (125B) and on another end to the flow focusing junction (134);
a second channel (132B) connected on one end to the second inlet (125B) and on another end to the flow focusing junction (124);
a third channel (133) connected on one end to the first inlet (111B) and on another end to the flow focusing junction (134);
wherein the bubble formation channel (135) is connected to the flow focusing junction (134);
wherein the flow focusing junction (134) is configured to receive a flow of said liquid via the first and second channels from two opposing directions that impinges in a perpendicular manner onto a flow of the second pressurized gas received via the third channel, wherein the flow of the second pressurized gas is directed from the third channel into the bubble formation channel.

9. The cartridge (100) according to any of the previous claims, further comprising an outlet (113) formed in the cartridge body (101) for outputting the liquid and second pressurized gas mixed by the mixing unit, wherein the outlet (113) is preferably formed to allow a Luer taper connection with a syringe.

10. The cartridge (100) according to claim 9, further comprising a buffer reservoir (140) formed in the cartridge body (101) and arranged in between the mixing unit and the outlet (113), said buffer reservoir (140) having a capacity that exceeds the volume of liquid held in the fluid storage system, wherein the buffer reservoir (140) is preferably vertically elongated when the cartridge (100) is held in an orientation in which a normal to the fluid channel plane is horizontal, and/or wherein the buffer reservoir (140) is preferably transparent.

11. The cartridge (100) according to claim 10, further comprising a venting hole (112) formed in the cartridge body (101) that is in fluid communication with the buffer reservoir (140);
wherein the cartridge (100) preferably comprises:
a fluid channel formed in the cartridge body (101) in between the one or more gas inlets (110, 111) and the mixing unit;
a fluid channel formed in the cartridge body (101) in between the one or more gas inlets (110, 111) and the fluid storage system;
a fluid channel formed in the cartridge body (101) in between the fluid storage system and the mixing unit;
a fluid channel formed in the cartridge body (101) in between the mixing unit and the buffer reservoir (140); and
a fluid channel formed in the cartridge body (101) in between the buffer reservoir (140) and the venting hole (112);
wherein the fluid channels extend essentially in a same fluid channel plane;
wherein the cartridge (100) is preferably configured to be in a first orientation during said mixing, wherein, in the first orientation:
a normal to the fluid channel plane is substantially horizontal;
the mixing unit is positioned at a lower position relative to the fluid storage system and relative to the gas inlet through which the second pressurized gas is received;
the fluid channel between the mixing unit and the buffer reservoir (140) outputs in the buffer reservoir (140) at a lower end of the buffer reservoir (140), preferably at or close to a lowest point of the buffer reservoir (140);
wherein the cartridge (100) is preferably configured to be in a second orientation during storage, wherein, in the second orientation:
a normal to the fluid channel plane is substantially vertical;
the fluid channel between the mixing unit and the buffer reservoir (140) outputs in the buffer reservoir (140) at a position above a liquid-air interface in the buffer reservoir (140) to prevent liquid outputted by the mixing unit into the buffer reservoir (140) to flow back into the fluid channel between the mixing unit and the buffer reservoir (140) when the cartridge (100) is in the second orientation, wherein the fluid channel between the venting hole and the buffer reservoir (140) preferably outputs in the buffer reservoir (140) at a position above the liquid-air interface when the cartridge (100) is in the second orientation.

12. The cartridge (100) according to any of the previous claims, further comprising filters arranged in each of said one or more gas inlets (110, 111) formed in the cartridge body (101), and, when applicable, in the venting hole, wherein at least one filter preferably comprises a filter membrane (115), a first filter support (116), and a second filter support (117), wherein the first and second filter supports are fixedly attached to or integrally formed with the cartridge body (101) and wherein the filter membrane is arranged in between the first and second filter supports.

13. The cartridge (100) according to any of the previous claims, wherein the cartridge (100) has been formed by fixedly attaching a first cartridge part and a second cartridge part using monolithic bonding, for example using ultrasonic welding, each cartridge part comprising a base layer;
wherein, prior to said fixedly attaching, one of the first and second cartridge part comprised ridges and/or protruding portions extending from the base layer that were configured to cooperate with protruding portions and/or ridges extending from the base layer of the other of the first and second cartridge part during ultrasonic welding as a result of which the protruding portions became integrally connected to the corresponding ridges;
the integrally connected ridges and protruding portions, and the base layer of the first and second cartridge parts together defining at least one of the mixing unit, the fluid storage system, the one or more gas inlets (110, 111), the outlet, the buffer reservoir (140), and the fluid channels for connecting them;
wherein the first and second cartridge parts are preferably made, using injection molding, from one or more materials from the group of thermoplastic materials such as polycarbonate.

14. A cartridge system, comprising:
a cartridge (100) as defined in any of the previous claims;
a device (200) comprising a housing with an opening (202) in which the cartridge (100) can be releasably inserted;
wherein the device (200) comprises one or more nozzles (242A, 242B) for inserting one or more pressurized gases into the one or more gas inlets (110, 111), for the purpose of said mixing by the mixing unit of the cartridge (100);
wherein the device (200) preferably further comprises an engaging unit (241) for engaging the sealed off container (120) arranged in the storage chamber (122) of the fluid storage unit for the purpose of causing the sealed off container (120) to break, rupture, or to become cut or pierced.

## Patentansprüche

1. Kartusche (100), umfassend:
einen Kartuschenkörper (101);
einen oder mehrere Gaseinlässe (110, 111), die in dem Kartuschenkörper (101) gebildet sind, konfiguriert um ein oder mehrere unter Druck stehende Gase aufzunehmen;
ein Flüssigkeitsspeichersystem, gebildet in dem Kartuschenkörper (101) und umfassend eine Flüssigkeitsspeichereinheit, die einen Einlass der Flüssigkeitsspeichereinheit, die mit einem ersten Gaseinlass unter den einen oder mehreren Gaseinlässen (110, 111) verbundenen ist, und einen Auslass der Flüssigkeitsspeichereinheit einschließt, wobei die Flüssigkeitsspeichereinheit konfiguriert ist, um eine Flüssigkeit zu halten und die Flüssigkeit durch ihren Auslass der Flüssigkeitsspeichereinheit in Antwort auf ein erstes unter Druck stehendes Gas aus dem einen oder den mehreren unter Druck stehenden Gasen, das ihr unter Verwendung des ersten unter Druck stehenden Gases als Treibmittel durch den ersten Gaseinlass zugeführt wird, auszugeben; und
eine Mischeinheit, die in dem Kartuschenkörper (101) angeordnet oder daran montiert ist und unter Verwendung eines in dem Kartuschenkörper (101) gebildeten Flüssigkeitskanals (125A) in Fluidverbindung mit der Flüssigkeitsspeichereinheit steht, wobei die Mischeinheit konfiguriert ist, um die aus der Flüssigkeitsspeichereinheit ausgegebene Flüssigkeit mit einem zweiten unter Druck stehenden Gas aus dem einen oder den mehreren unter Druck stehenden Gasen zu mischen, wobei:
in dem Fall, dass das erste unter Druck stehende Gas das gleiche Gas wie das zweite unter Druck stehende Gas ist, das zweite unter Druck stehende Gas durch den ersten Gaseinlass oder durch einen zweiten Gaseinlass unter dem einen oder den mehreren Gaseinlässen (110, 111) aufgenommen wird, und
in dem Fall, dass sich das erste unter Druck stehende Gas von dem zweiten unter Druck stehenden Gas unterscheidet, das zweite unter Druck stehende Gas durch einen zweiten Gaseinlass unter dem einen oder den mehreren Gaseinlässen (110, 111) aufgenommen wird,
**dadurch gekennzeichnet, dass** die Flüssigkeitsspeichereinheit umfasst:
eine Speicherkammer (122), konfiguriert, um einen abgedichteten Behälter (120) aufzunehmen, in dem die Flüssigkeit steril und abgedichtet aufbewahrt wird; und
einen Flüssigkeitsbehälter (125) in Fluidverbindung mit der Speicherkammer (122), wobei der Einlass der Flüssigkeitsspeichereinheit entweder mit der Speicherkammer (122) oder dem Flüssigkeitsbehälter (125) verbunden ist und wobei der Auslass der Flüssigkeitsspeichereinheit mit dem Flüssigkeitsbehälter (125) verbunden ist;
wobei der Flüssigkeitsbehälter (125) konfiguriert ist, um Flüssigkeit zu sammeln, die aus dem abgedichteten Behälter (120) freigesetzt wird, nachdem der abgedichtete Behälter (120) zerbrochen, zerrissen, geschnitten oder durchstochen wurde.

2. Kartusche (100) nach Anspruch 1, wobei die in dem Flüssigkeitsspeichersystem enthaltene Flüssigkeit zur intrakorporalen Verwendung bei einem Menschen oder einem Tier bestimmt ist, beispielsweise zur intravenösen Verwendung oder zur Verwendung in einem Hohlraum, wobei die Flüssigkeit vorzugsweise umfasst oder verwendet wird um ein diagnostisches Mittel zu bilden, wie ein Kontrastmittel für die medizinische Bildgebung, oder ein therapeutisches Mittel, wie ein Medikament.

3. Kartusche (100) nach einem der vorstehenden Ansprüche, wobei der abgedichtete Behälter (120) eine Blisterverpackung umfasst und/oder wobei der abgedichtete Behälter (120) fest in der Speicherkammer (122) gehalten wird, beispielsweise mittels eines Klebstoffs.

4. Kartusche (100) nach einem der vorstehenden Ansprüche, wobei der Flüssigkeitsbehälter (125) als Teil der Speicherkammer (122) gebildet ist, oder wobei die Flüssigkeitsspeichereinheit einen in dem Kartuschenkörper (101) gebildeten Flüssigkeitskanal umfasst, der die Speicherkammer (122) und den Flüssigkeitsbehälter (125) verbindet.

5. Kartusche (100) nach einem der vorstehenden Ansprüche, wobei die Speicherkammer (122) eine Stützfläche zum Stützen des abgedichteten Behälters (120) und mindestens einen vorstehenden Stift oder eine vorstehende Nadel umfasst, der bzw. die sich in Richtung des abgedichteten Behälters (120) erstreckt, um den abgedichteten Behälter zu durchstechen, wenn eine ausreichende Kraft auf den abgedichteten Behälter (120) ausgeübt wird, wobei die Flüssigkeitsspeichereinheit vorzugsweise mit einem Schutzring bereitgestellt ist, der von dem Kartuschenkörper (101) weiter als der abgedichtete Behälter (120) absteht, wenn er in die Speicherkammer (122) eingesetzt ist.

6. Kartusche (100) nach einem der vorstehenden Ansprüche, wobei die Mischeinheit konfiguriert ist, um die von dem Flüssigkeitsspeichersystem erhaltene Flüssigkeit mit dem zweiten unter Druck stehenden Gas zu mischen, wobei die Mischeinheit eine Mikrofluidikvorrichtung (130) umfasst, konfiguriert, um in der Flüssigkeit Mikrobläschen zu erzeugen, die mit dem zweiten unter Druck stehenden Gas gefüllt sind.

7. Kartusche (100) nach Anspruch 6, wobei die Mikrofluidikvorrichtung (130) konfiguriert ist, um Mikrobläschen mit einem Durchmesser von weniger als 10 Mikrometer und vorzugsweise in einem Bereich von 2-5 Mikrometer zu erzeugen; und/oder
wobei das zweite unter Druck stehende Gas ein Gas aus der Gruppe bestehend aus SF₆, N₂, CO₂, O₂, H₂, He, Ar, Umgebungsluft und Perfluorkohlenstoffgasen wie CF₄, C₂F₆, C₂F₈, C₃F₆, C₃F₈, C₄F₆, C₄F₈, C₄F₁₀, C₅F₁₀, C₅F₁₂ und Mischungen davon umfasst, und/oder
wobei die aus dem Flüssigkeitsspeichersystem aufgenommene Flüssigkeit eine Flüssigkeit aus der Gruppe bestehend aus Wasser, Dispersion von Lipiden, wie Phospholipiden, oder Proteinen in einer wässrigen Lösung, pharmazeutisch wirksamen Bestandteilen und Alkoholen umfasst.

8. Kartusche (100) nach einem der Ansprüche 6 bis 7, wobei die Mikrofluidikvorrichtung (130) umfasst:
einen ersten Einlass (111B) zum Aufnehmen des zweiten unter Druck stehenden Gases;
einen zweiten Einlass (125B) zum Aufnehmen der Flüssigkeit;
einen Blasenbildungskanal (135) zum Erzeugen der Mikroblasen basierend auf einem Strom des zweiten unter Druck stehenden Gases, das durch den ersten Einlass aufgenommen wird, und einem Strom der Flüssigkeit, die durch den zweiten Einlass aufgenommen wird;
wobei vorzugsweise der Kartuschenkörper (101) eine erste Öffnung, eine zweite Öffnung und eine dritte Öffnung umfasst, wobei die erste Öffnung in Fluidverbindung mit dem Gaseinlass unter dem einen oder den mehreren Gaseinlässen steht, der das zweite unter Druck stehende Gas aufnimmt, und wobei die zweite Öffnung in Fluidverbindung mit dem Flüssigkeitsspeichersystem zum Zweck des Aufnehmens der Flüssigkeit steht, wobei die Mikrofluidikvorrichtung (130) relativ zu dem Kartuschenkörper (101) positioniert ist, so dass die erste Öffnung mit dem ersten Einlass der Mikrofluidikvorrichtung (130), die zweite Öffnung mit dem zweiten Einlass der Mikrofluidikvorrichtung (130), und die dritte Öffnung mit einem Auslass der Mikrofluidikvorrichtung (130) ausgerichtet ist, wobei die Mikrofluidikvorrichtung (130) unter Verwendung eines Klebstoffs oder einer monolithischen Bindung fest mit dem Kartuschenkörper (101) verbunden ist, oder wobei die Mikrofluidikvorrichtung (130) integral mit dem Kartuschenkörper (101) gebildet ist;
wobei die Mikrofluidikvorrichtung (130) vorzugsweise umfasst:
einen Flussfokussierungsübergang (134);
einen ersten Kanal (132A), der an einem Ende mit dem zweiten Einlass (125B) und an einem anderen Ende mit dem Flussfokussierungsübergang (134) verbunden ist;
einen zweiten Kanal (132B), der an einem Ende mit dem zweiten Einlass (125B) und an einem anderen Ende mit dem Flussfokussierungsübergang (124) verbunden ist;
einen dritten Kanal (133), der an einem Ende mit dem ersten Einlass (111B) und an einem anderen Ende mit dem Flussfokussierungsübergang (134) verbunden ist;
wobei der Blasenbildungskanal (135) mit dem Flussfokussierungsübergang (134) verbunden ist;
wobei der Flussfokussierungsübergang (134) konfiguriert ist, um einen Strom der Flüssigkeit über den ersten und zweiten Kanal aus zwei entgegengesetzten Richtungen aufzunehmen, der in senkrechter Weise auf einen Strom des zweiten unter Druck stehenden Gases auftrifft, das über den dritten Kanal aufgenommen wird, wobei der Strom des zweiten unter Druck stehenden Gases von dem dritten Kanal in den Blasenbildungskanal geleitet wird.

9. Kartusche (100) nach einem der vorstehenden Ansprüche, ferner umfassend einen in dem Kartuschenkörper (101) gebildeten Auslass (113) zum Ausgeben der Flüssigkeit und des zweiten unter Druck stehenden Gases, gemischt durch die Mischeinheit, wobei der Auslass (113) vorzugsweise gebildet ist, um eine Luer-Kegel-Verbindung mit einer Spritze zu ermöglichen.

10. Kartusche (100) nach Anspruch 9, ferner umfassend einen Pufferbehälter (140), gebildet in dem Kartuschenkörper (101) und angeordnet zwischen der Mischeinheit und dem Auslass (113), wobei der Pufferbehälter (140) ein Fassungsvermögen hat, das das in dem Flüssigkeitsspeichersystem gehaltene Flüssigkeitsvolumen übersteigt, wobei der Pufferbehälter (140) vorzugsweise vertikal verlängert ist, wenn die Kartusche (100) in einer Ausrichtung gehalten wird, in der eine Normale zu der Fluidkanalebene horizontal ist, und/oder wobei der Pufferbehälter (140) vorzugsweise transparent ist.

11. Kartusche (100) nach Anspruch 10, ferner umfassend ein in dem Kartuschenkörper (101) gebildetes Entlüftungsloch (112), das in Fluidverbindung mit dem Pufferbehälter (140) ist;
wobei die Kartusche (100) vorzugsweise umfasst:
einen in dem Kartuschenkörper (101) zwischen dem einen oder den mehreren Gaseinlässen (110, 111) und der Mischeinheit gebildeten Fluidkanal;
einen in dem Kartuschenkörper (101) zwischen dem einen oder den mehreren Gaseinlässen (110, 111) und dem Flüssigkeitsspeichersystem gebildeten Fluidkanal;
einen in dem Kartuschenkörper (101) zwischen dem Flüssigkeitsspeichersystem und der Mischeinheit gebildeten Fluidkanal;
einen in dem Kartuschenkörper (101) zwischen der Mischeinheit und dem Pufferbehälter (140) gebildeten Fluidkanal; und
einen in dem Kartuschenkörper (101) zwischen dem Pufferbehälter (140) und dem Entlüftungsloch (112) gebildeten Fluidkanal;
wobei sich die Fluidkanäle im Wesentlichen in einer gleichen Fluidkanalebene erstrecken;
wobei die Kartusche (100) vorzugsweise konfiguriert ist, um sich während des Mischens in einer ersten Ausrichtung zu befinden, wobei in der ersten Ausrichtung:
eine Normale zu der Fluidkanalebene im Wesentlichen horizontal ist;
die Mischeinheit in einer unteren Position relativ zu dem Flüssigkeitsspeichersystem und relativ zu dem Gaseinlass, durch den das zweite unter Druck stehende Gas aufgenommen wird, positioniert ist;
der Fluidkanal zwischen der Mischeinheit und dem Pufferbehälter (140) in den Pufferbehälter (140) an einem unteren Ende des Pufferbehälters (140), vorzugsweise an oder nahe einem tiefsten Punkt des Pufferbehälters (140), mündet;
wobei die Kartusche (100) vorzugsweise konfiguriert ist, um sich während der Lagerung in einer zweiten Ausrichtung zu befinden, wobei in der zweiten Ausrichtung:
eine Normale zu der Fluidkanalebene im Wesentlichen vertikal ist;
der Fluidkanal zwischen der Mischeinheit und dem Pufferbehälter (140) in den Pufferbehälter (140) an einer Position über einer Flüssigkeits-Luft-Grenzfläche in dem Pufferbehälter (140) mündet, um zu verhindern, dass von der Mischeinheit in den Pufferbehälter (140) ausgegebene Flüssigkeit in den Fluidkanal zwischen der Mischeinheit und dem Pufferbehälter (140) zurückfließt, wenn sich die Kartusche (100) in der zweiten Ausrichtung befindet, wobei der Fluidkanal zwischen dem Entlüftungsloch und dem Pufferbehälter (140) vorzugsweise in dem Pufferbehälter (140) an einer Position über der Flüssigkeits-Luft-Grenzfläche mündet, wenn sich die Kartusche (100) in der zweiten Ausrichtung befindet.

12. Kartusche (100) nach einem der vorstehenden Ansprüche, ferner umfassend Filter, die in jedem der einen oder mehreren Gaseinlässen (110, 111) angeordnet sind, die in dem Kartuschenkörper (101) und, falls zutreffend, in dem Entlüftungloch gebildet sind, wobei mindestens ein Filter vorzugsweise eine Filtermembran (115), eine erste Filterstütze (116) und eine zweite Filterstütze (117) umfasst, wobei die erste und die zweite Filterstütze fest an dem Kartuschenkörper (101) angebracht oder integral mit diesem ausgebildet sind und wobei die Filtermembran zwischen der ersten und der zweiten Filterstütze angeordnet ist.

13. Kartusche (100) nach einem der vorstehenden Ansprüche, wobei die Kartusche (100) durch festes Anbringen eines ersten Kartuscheteils und eines zweiten Kartuscheteils unter Verwendung von monolithischem Kleben, beispielsweise unter Verwendung von Ultraschallschweißen, gebildet wurde, wobei jeder Kartuscheteil eine Basisschicht umfasst;
Wobei, vor dem festen Anbringen, eines von dem ersten und dem zweiten Kartuscheteil Rippen und/oder vorstehende Abschnitte umfasst, die sich von der Basisschicht erstrecken, konfiguriert sind, um während des Ultraschallschweißens mit vorstehenden Abschnitten und/oder Rippen zusammenzuwirken, die sich von der Basisschicht des anderen von dem ersten und dem zweiten Kartuscheteil erstrecken, wodurch die vorstehenden Abschnitte integral mit den entsprechenden Rippen verbunden wurden;
die integral verbundenen Rippen und vorstehenden Abschnitte und die Basisschicht der ersten und zweiten Kartuscheteile zusammen mindestens eine von der Mischeinheit, dem Flüssigkeitsspeichersystem, dem einen oder den mehreren Gaseinlässen (110, 111), dem Auslass, dem Pufferspeicher (140) und den Flüssigkeitskanälen zu deren Verbindung definieren;
wobei die ersten und zweiten Kartuscheteile vorzugsweise unter Verwendung von Spritzguss aus einem oder mehreren Materialien aus der Gruppe der thermoplastischen Materialien, wie etwa Polycarbonat, hergestellt sind.

14. Kartuschensystem, umfassend:
eine Kartusche (100), wie in einem der vorstehenden Ansprüche definiert;
eine Vorrichtung (200), umfassend ein Gehäuse mit einer Öffnung (202), in die die Kartusche (100) lösbar eingesetzt werden kann;
wobei die Vorrichtung (200) eine oder mehrere Düsen (242A, 242B) zum Einführen eines oder mehrerer unter Druck stehender Gase in den einen oder die mehreren Gaseinlässe (110, 111) umfasst, zum Zweck des Mischens durch die Mischeinheit der Kartusche (100);
wobei die Vorrichtung (200) vorzugsweise ferner eine Eingriffseinheit (241) zum Eingreifen in den abgedichteten Behälter (120) umfasst, der in der Speicherkammer (122) der Flüssigkeitsspeichereinheit angeordnet ist, um Zweck, dass der abgedichtete Behälter (120) zerbricht, zerreißt oder durchschnitten oder durchstochen wird.

## Revendications

1. Cartouche (100), comprenant :
un corps de cartouche (101) ;
une ou plusieurs entrées de gaz (110, 111) formées dans le corps de cartouche (101) configurées pour recevoir un ou plusieurs gaz sous pression, respectivement ;
un système de stockage de fluide formé dans le corps de cartouche (101) et comprenant une unité de stockage de fluide qui comporte une entrée d'unité de stockage de fluide qui est connectée à une première entrée de gaz parmi les une ou plusieurs entrées de gaz (110, 111) et une sortie d'unité de stockage de fluide, dans laquelle l'unité de stockage de fluide est configurée pour contenir un liquide et pour faire sortir ledit liquide à travers sa sortie **d'unité** de stockage de fluide en réponse à un premier gaz sous pression parmi les un ou plusieurs gaz sous pression qui est fourni à celle-ci à travers ladite première entrée de gaz en utilisant le premier gaz sous pression comme propulseur ; et
une unité de mélange agencée dans le corps de cartouche (101), ou montée sur celui-ci, et qui est en communication fluidique avec l'unité de stockage de fluide à l'aide d'un canal de fluide (125A) formé dans le corps de cartouche (101), ladite unité de mélange étant configurée pour mélanger le liquide sorti de l'unité de stockage de fluide avec un second gaz sous pression parmi les un ou plusieurs gaz sous pression, dans laquelle :
dans le cas où le premier gaz sous pression est le même gaz que le second gaz sous pression, le second gaz sous pression est reçu à travers la première entrée de gaz ou à travers une seconde entrée de gaz parmi les une ou plusieurs entrées de gaz (110, 111), et
dans le cas où le premier gaz sous pression est différent du second gaz sous pression, le second gaz sous pression est reçu à travers une seconde entrée de gaz parmi les une ou plusieurs entrées de gaz (110, 111) ;
**caractérisée en ce que** l'unité de stockage de fluide comprend :
une chambre de stockage (122) configurée pour recevoir un récipient étanchéifié (120) dans lequel le liquide est contenu d'une manière stérile et étanchéifiée ; et
un réservoir de liquide (125) en communication fluidique avec la chambre de stockage (122), dans laquelle l'entrée d'unité de stockage de fluide est connectée à l'un de la chambre de stockage (122) et du réservoir de liquide (125) et dans laquelle la sortie d'unité de stockage de fluide est connectée au réservoir de liquide (125) ;
dans laquelle le réservoir de liquide (125) est configuré pour collecter un liquide qui est libéré du récipient étanchéfié (120) après que le récipient étanchéfié (120) a été cassé, rompu, coupé ou percé.

2. Cartouche (100) selon la revendication 1, dans laquelle le liquide contenu dans le système de stockage de fluide est destiné à une utilisation intra-corporelle chez un être humain ou un animal, par exemple pour une utilisation intraveineuse ou une utilisation intra-cavité, dans laquelle le liquide comprend de préférence un agent diagnostique, ou est utilisé pour former celui-ci, tel qu'un agent de contraste pour imagerie médicale, ou un agent thérapeutique, tel qu'un médicament.

3. Cartouche (100) selon l'une quelconque des revendications précédentes, dans laquelle le récipient étanchéifié (120) comprend un emballage blister et/ou dans laquelle le récipient étanchéifié (120) est maintenu de manière fixe dans la chambre de stockage (122), par exemple au moyen d'un adhésif.

4. Cartouche (100) selon l'une quelconque des revendications précédentes, dans laquelle le réservoir de liquide (125) est formé en tant que partie de la chambre de stockage (122), ou dans laquelle ladite unité de stockage de fluide comprend un canal de fluide formé dans le corps de cartouche (101) reliant la chambre de stockage (122) et le réservoir de liquide (125).

5. Cartouche (100) selon l'une quelconque des revendications précédentes, dans laquelle la chambre de stockage (122) comprend une surface de support pour supporter le récipient étanchéifié (120) et au moins une goupille ou aiguille en saillie s'étendant vers le récipient étanchéifié (120) dans le but de percer le récipient étanchéifié si une force suffisante est exercée sur le récipient étanchéifié (120), dans laquelle l'unité de stockage de fluide est de préférence pourvue d'une bague de protection qui fait saillie à l'opposé du corps de cartouche (101) de manière plus éloignée que le récipient étanchéifié (120) lorsqu'elle est placée dans la chambre de stockage (122).

6. Cartouche (100) selon l'une quelconque des revendications précédentes, dans laquelle l'unité de mélange est configurée pour mélanger le liquide reçu du système de stockage de fluide avec le second gaz sous pression, ladite unité de mélange comprenant un dispositif micro-fluidique (130) configuré pour générer des micro-bulles à l'intérieur dudit liquide qui sont remplies du second gaz sous pression.

7. Cartouche (100) selon la revendication 6, dans laquelle le dispositif micro-fluidique (130) est configuré pour générer des micro-bulles ayant un diamètre inférieur à 10 micromètres, et de préférence dans une plage de 2 à 5 micromètres ; et/ou
dans laquelle le second gaz sous pression comprend un gaz du groupe consistant en SF₆, N₂, CO₂, O₂, H₂, He, Ar, air ambiant, et des gaz perfluorocarbonés, tels que CF₄, C₂F₆, C₂F₈, C₃F₆, C₃Fₑ, C₄F₆, C₄F₈, C₄F₁₀, C₅F₁₀, C₅F₁₂ et leurs mélanges ; et/ou
dans laquelle ledit liquide reçu du système de stockage de fluide comprend un liquide du groupe consistant en de l'eau, dispersion de lipides, tels que des phospholipides, ou de protéines dans une solution aqueuse, ingrédients pharmaceutiques actifs, et alcools.

8. Cartouche (100) selon l'une quelconque des revendications 6 à 7, dans laquelle le dispositif micro-fluidique (130) comprend :
une première entrée (111B) pour recevoir le second gaz sous pression ;
une seconde entrée (125B) pour recevoir ledit liquide ;
un canal de formation de bulles (135) pour générer les micro-bulles sur la base d'un flux du second gaz sous pression reçu à travers la première entrée et d'un flux du liquide reçu à travers la seconde entrée ;
dans laquelle, de préférence, le corps de cartouche (101) comprend une première ouverture, une deuxième ouverture, et une troisième ouverture, dans laquelle la première ouverture est en communication fluidique avec l'entrée de gaz parmi les une ou plusieurs entrées de gaz qui reçoit le second gaz sous pression, et dans laquelle la deuxième ouverture est en communication fluidique avec le système de stockage de fluide dans le but de recevoir le liquide, dans laquelle le dispositif micro-fluidique (130) est positionné par rapport au corps de cartouche (101) de telle sorte que la première ouverture est alignée avec la première entrée du dispositif micro-fluidique (130), la deuxième ouverture avec la seconde entrée du dispositif micro-fluidique (130), et la troisième ouverture avec une sortie du dispositif micro-fluidique (130), dans laquelle le dispositif micro-fluidique (130) est connecté de manière fixe au corps de cartouche (101) à l'aide d'un adhésif ou d'une liaison monolithique, ou dans laquelle le dispositif micro-fluidique (130) est formé d'un seul tenant avec le corps de cartouche (101) ;
dans laquelle le dispositif micro-fluidique (130) comprend de préférence :
une jonction de focalisation de flux (134) ;
un premier canal (132A) connecté sur une extrémité à la seconde entrée (125B) et sur une autre extrémité à la jonction de focalisation de flux (134) ;
un deuxième canal (132B) connecté sur une extrémité à la seconde entrée (125B) et sur une autre extrémité à la jonction de focalisation de flux (124) ;
un troisième canal (133) connecté sur une extrémité à la première entrée (111B) et sur une autre extrémité à la jonction de focalisation de flux (134) ;
dans laquelle le canal de formation de bulles (135) est connecté à la jonction de focalisation de flux (134) ;
dans laquelle la jonction de focalisation de flux (134) est configurée pour recevoir un flux dudit liquide via les premier et deuxième canaux à partir de deux directions opposées qui influence de manière perpendiculaire un flux du second gaz sous pression reçu via le troisième canal, dans laquelle le flux du second gaz sous pression est dirigé à partir du troisième canal dans le canal de formation de bulles.

9. Cartouche (100) selon l'une quelconque des revendications précédentes, comprenant en outre une sortie (113) formée dans le corps de cartouche (101) pour faire sortir le liquide et le second gaz sous pression mélangés par l'unité de mélange, dans laquelle la sortie (113) est de préférence formée pour permettre un raccord conique Luer avec une seringue.

10. Cartouche (100) selon la revendication 9, comprenant en outre un réservoir tampon (140) formé dans le corps de cartouche (101) et agencé entre l'unité de mélange et la sortie (113), ledit réservoir tampon (140) ayant une capacité qui dépasse le volume de liquide contenu dans le système de stockage de fluide, dans laquelle le réservoir tampon (140) est de préférence allongé verticalement lorsque la cartouche (100) est maintenue dans une orientation dans laquelle une normale au plan de canaux de fluide est horizontale, et/ou dans laquelle le réservoir tampon (140) est de préférence transparent.

11. Cartouche (100) selon la revendication 10, comprenant en outre un orifice d'aération (112) formé dans le corps de cartouche (101) qui est en communication fluidique avec le réservoir tampon (140) ;
dans laquelle la cartouche (100) comprend de préférence :
un canal de fluide formé dans le corps de cartouche (101) entre les une ou plusieurs entrées de gaz (110, 111) et l'unité de mélange ;
un canal de fluide formé dans le corps de cartouche (101) entre les une ou plusieurs entrées de gaz (110, 111) et le système de stockage de fluide ;
un canal de fluide formé dans le corps de cartouche (101) entre le système de stockage de fluide et l'unité de mélange ;
un canal de fluide formé dans le corps de cartouche (101) entre l'unité de mélange et le réservoir tampon (140) ; et
un canal de fluide formé dans le corps de cartouche (101) entre le réservoir tampon (140) et l'orifice d'aération (112) ;
dans laquelle les canaux de fluide s'étendent essentiellement dans un même plan de canaux de fluide ;
dans laquelle la cartouche (100) est de préférence configurée pour être dans une première orientation pendant ledit mélange, dans laquelle, dans la première orientation :
une normale au plan de canaux de fluide est sensiblement horizontale ;
l'unité de mélange est positionnée à une position inférieure par rapport au système de stockage de fluide et par rapport à l'entrée de gaz à travers laquelle le second gaz sous pression est reçu ;
le canal de fluide entre l'unité de mélange et le réservoir tampon (140) sort dans le réservoir tampon (140) à une extrémité inférieure du réservoir tampon (140), de préférence au niveau ou à proximité d'un point le plus bas du réservoir tampon (140) ;
dans laquelle la cartouche (100) est de préférence configurée pour être dans une seconde orientation pendant le stockage, dans laquelle, dans la seconde orientation :
une normale au plan de canaux de fluide est sensiblement verticale ;
le canal de fluide entre l'unité de mélange et le réservoir tampon (140) sort dans le réservoir tampon (140) à une position au-dessus d'une interface liquide-air dans le réservoir tampon (140) pour empêcher le liquide sorti par l'unité de mélange dans le réservoir tampon (140) de refluer dans le canal de fluide entre l'unité de mélange et le réservoir tampon (140) lorsque la cartouche (100) est dans la seconde orientation, dans laquelle le canal de fluide entre l'orifice d'aération et le réservoir tampon (140) sort de préférence dans le réservoir tampon (140) à une position au-dessus de l'interface liquide-air lorsque la cartouche (100) est dans la seconde orientation.

12. Cartouche (100) selon l'une quelconque des revendications précédentes, comprenant en outre des filtres agencés dans chacune desdites une ou plusieurs entrées de gaz (110, 111) formées dans le corps de cartouche (101), et, le cas échéant, dans l'orifice d'aération, dans laquelle au moins un filtre comprend de préférence une membrane filtrante (115), un premier support de filtre (116), et un second support de filtre (117), dans laquelle les premier et second supports de filtre sont attachés de manière fixe au corps de cartouche (101), ou formés d'un seul tenant avec celui-ci, et dans laquelle la membrane filtrante est agencée entre les premier et second supports de filtre.

13. Cartouche (100) selon l'une quelconque des revendications précédentes, dans laquelle la cartouche (100) a été formée en attachant de manière fixe une première partie de cartouche et une seconde partie de cartouche à l'aide d'une liaison monolithique, par exemple à l'aide d'un soudage par ultrasons, chaque partie de cartouche comprenant une couche de base ;
dans laquelle, avant de l'attacher de manière fixe, l'une des première et seconde parties de cartouche comprenait des arêtes et/ou des portions en saillie s'étendant à partir de la couche de base qui étaient configurées pour coopérer avec des portions en saillie et/ou des arêtes s'étendant à partir de la couche de base de l'autre des première et seconde parties de cartouche pendant le soudage par ultrasons dont le résultat est que les portions en saillie sont devenues connectées d'un seul tenant aux arêtes correspondantes ;
les arêtes et les portions en saillie connectées d'un seul tenant, et la couche de base des première et seconde parties de cartouche définissant ensemble au moins l'un de l'unité de mélange, du système de stockage de fluide, des une ou plusieurs entrées de gaz (110, 111), de la sortie, du réservoir tampon (140), et des canaux de fluide pour les connecter ;
dans laquelle les première et seconde parties de cartouche sont de préférence réalisées, à l'aide d'un moulage par injection, à partir d'un ou plusieurs matériaux du groupe de matériaux thermoplastiques tels que du polycarbonate.

14. Système de cartouche, comprenant :
une cartouche (100) selon l'une quelconque des revendications précédentes ;
un dispositif (200) comprenant un logement avec une ouverture (202) dans laquelle la cartouche (100) peut être insérée de manière libérable ;
dans lequel le dispositif (200) comprend une ou plusieurs buses (242A, 242B) pour insérer un ou plusieurs gaz sous pression dans les une ou plusieurs entrées de gaz (110, 111), dans le but dudit mélange par l'unité de mélange de la cartouche (100) ;
dans lequel le dispositif (200) comprend de préférence en outre une unité de mise en prise (241) pour mettre en prise le récipient étanchéifié (120) agencé dans la chambre de stockage (122) de l'unité de stockage de fluide afin de provoquer la cassure, la rupture, ou la coupure ou la perforation du récipient étanchéifié (120).
